# EUROPEAN PATENT APPLICATION

(11) **EP 3 916 015 A1**
(43) Date of publication of application: **01.12.2021**
(21) Application number: 20305561.1
(22) Date of filing: 28.05.2020
(51) Int. Cl.: C07K 16/28, A61P 25/18, A61K 39/00

(54) **ANTI-HERV-W ENVELOPE PROTEIN ANTIBODY FOR USE IN THE TREATMENT OF PSYCHOTIC DISEASES**

(71) Applicant: Geneuro SA, 1228 Plan-Les-Ouates (CH); Fondamental, 94000 Creteil (FR)
(72) Inventor: The designation of the inventor has not yet been filed
(74) Representative: Plasseraud IP

(57) **Abstract**

The present invention relates to an antibody directed against HERV-W envelope protein (ENV) for use in the treatment of a group of patients suffering from a psychotic disease and having a high level for a cytokine in a body fluid sample.

SEQUENCE LISTING

<110>  GENEURO SA
       FONDAMENTAL

<120>  Anti-HERV-W envelope protein antibody for use in the treatment of
        psychotic diseases

<130>  BEP191154 EP

<160>  13

<170>  PatentIn version 3.5

<210>  1
<211>  11
<212>  PRT
<213>  artificial sequence

<220>
<223>  CDR-VL1

<400>  1

Lys Ser Leu Leu His Ser Lys Gly Ile Thr Tyr
1               5                   10


<210>  2
<211>  3
<212>  PRT
<213>  artificial sequence

<220>
<223>  CDR-VL2

<400>  2

Gln Met Ser
1


<210>  3
<211>  9
<212>  PRT
<213>  artificial sequence

<220>
<223>  CDR-VL3

<400>  3

Ala Gln Asn Leu Glu Leu Pro Trp Thr
1               5


<210>  4
<211>  8
<212>  PRT
<213>  artificial sequence

<220>
<223>  CDR-VH1

<400>  4

Gly Tyr Ser Phe Thr Ser Tyr Trp
1               5


<210>  5
<211>  9
<212>  PRT
<213>  artificial sequence

<220>
<223>  CDR-VH2

<400>  5

Ile Tyr Pro Gly Lys Ser Asp Thr Ser
1               5


<210>  6
<211>  9
<212>  PRT
<213>  artificial sequence

<220>
<223>  CDR-VH3

<400>  6

Thr Arg Thr Val Tyr Ala Met Asp Tyr
1               5


<210>  7
<211>  112
<212>  PRT
<213>  artificial sequence

<220>
<223>  VL

<400>  7

Asp Ile Val Met Thr Gln Ala Ala Phe Ser Asn Pro Val Thr Leu Gly
1               5                   10                  15


Thr Ser Ala Ser Ile Ser Cys Arg Ser Ser Lys Ser Leu Leu His Ser
            20                  25                  30


Lys Gly Ile Thr Tyr Leu Tyr Trp Phe Leu Gln Lys Pro Gly Gln Ser
        35                  40                  45


Pro Gln Leu Leu Ile Tyr Gln Met Ser His Leu Ala Ser Gly Val Pro
    50                  55                  60


Asp Arg Phe Ser Ser Ser Gly Ser Gly Thr Asp Phe Thr Leu Arg Ile
65                  70                  75                  80

Ser Arg Val Glu Ala Glu Asp Val Gly Val Tyr Tyr Cys Ala Gln Asn
                85                  90                  95

Leu Glu Leu Pro Trp Thr Phe Gly Gly Gly Thr Lys Leu Glu Ile Lys
            100                 105                 110

<210>  8
<211>  116
<212>  PRT
<213>  artificial sequence

<220>
<223>  VH

<400>  8

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Ser Phe Thr Ser Tyr
            20                  25                  30

Trp Met His Trp Ile Lys Gln Arg Pro Gly Gln Gly Leu Glu Trp Ile
        35                  40                  45

Gly Ala Ile Tyr Pro Gly Lys Ser Asp Thr Ser Tyr Asn Gln Lys Phe
    50                  55                  60

Lys Gly Lys Ala Lys Leu Thr Ala Val Thr Ser Ala Ser Thr Ala Tyr
65                  70                  75                  80

Met Glu Leu Thr Ser Leu Thr Asn Glu Asp Ser Ala Val Tyr Tyr Cys
                85                  90                  95

Thr Arg Thr Val Tyr Ala Met Asp Tyr Trp Gly Gln Gly Thr Ser Val
            100                 105                 110

Thr Val Ser Ser
        115

<210>  9
<211>  542
<212>  PRT
<213>  protein ENV of the Human endogenous retrovirus-W

<400>  9

Met Ala Leu Pro Tyr His Thr Phe Leu Phe Thr Val Leu Leu Pro Pro
1               5                   10                  15

Phe Ala Leu Thr Ala Pro Pro Pro Cys Cys Cys Thr Thr Ser Ser Ser
            20                  25                  30

Pro Tyr Gln Glu Phe Leu Trp Arg Thr Arg Leu Pro Gly Asn Ile Asp
        35                  40                  45

Ala Pro Ser Tyr Arg Ser Leu Ser Lys Gly Asn Ser Thr Phe Thr Ala
    50                  55                  60

His Thr His Met Pro Arg Asn Cys Tyr Asn Ser Ala Thr Leu Cys Met
65                  70                  75                  80

His Ala Asn Thr His Tyr Trp Thr Gly Lys Met Ile Asn Pro Ser Cys
                85                  90                  95

Pro Gly Gly Leu Gly Ala Thr Val Cys Trp Thr Tyr Phe Thr His Thr
            100                 105                 110

Ser Met Ser Asp Gly Gly Gly Ile Gln Gly Gln Ala Arg Glu Lys Gln
        115                 120                 125

Val Lys Glu Ala Ile Ser Gln Leu Thr Arg Gly His Ser Thr Pro Ser
    130                 135                 140

Pro Tyr Lys Gly Leu Val Leu Ser Lys Leu His Glu Thr Leu Arg Thr
145                 150                 155                 160

His Thr Arg Leu Val Ser Leu Phe Asn Thr Thr Leu Thr Arg Leu His
                165                 170                 175

Glu Val Ser Ala Gln Asn Pro Thr Asn Cys Trp Met Cys Leu Pro Leu
            180                 185                 190

His Phe Arg Pro Tyr Ile Ser Ile Pro Val Pro Glu Gln Trp Asn Asn
        195                 200                 205

Phe Ser Thr Glu Ile Asn Thr Thr Ser Val Leu Val Gly Pro Leu Val
    210                 215                 220

Ser Asn Leu Glu Ile Thr His Thr Ser Asn Leu Thr Cys Val Lys Phe
225                 230                 235                 240

Ser Asn Thr Ile Asp Thr Thr Ser Ser Gln Cys Ile Arg Trp Val Thr
                245                 250                 255

Pro Pro Thr Arg Ile Val Cys Leu Pro Ser Gly Ile Phe Phe Val Cys
            260                 265                 270

Gly Thr Ser Ala Tyr His Cys Leu Asn Gly Ser Ser Glu Ser Met Cys
        275                 280                 285

Phe Leu Ser Phe Leu Val Pro Pro Met Thr Ile Tyr Thr Glu Gln Asp
    290                 295                 300

Leu Tyr Asn His Val Val Pro Lys Pro His Asn Lys Arg Val Pro Ile
305                 310                 315                 320

Leu Pro Phe Val Ile Arg Ala Gly Val Leu Gly Arg Leu Gly Thr Gly
                325                 330                 335

Ile Gly Ser Ile Thr Thr Ser Thr Gln Phe Tyr Tyr Lys Leu Ser Gln
            340                 345                 350

Glu Ile Asn Gly Asp Met Glu Gln Val Thr Asp Ser Leu Val Thr Leu
        355                 360                 365

Gln Asp Gln Leu Asn Ser Leu Ala Ala Val Val Leu Gln Asn Arg Arg
    370                 375                 380

Ala Leu Asp Leu Leu Thr Ala Lys Arg Gly Gly Thr Cys Leu Phe Leu
385                 390                 395                 400

Gly Glu Glu Arg Cys Tyr Tyr Val Asn Gln Ser Arg Ile Val Thr Glu
                405                 410                 415

Lys Val Lys Glu Ile Arg Asp Arg Ile Gln Cys Arg Ala Glu Glu Leu
            420                 425                 430

Gln Asn Thr Glu Arg Trp Gly Leu Leu Ser Gln Trp Met Pro Trp Thr
        435                 440                 445

Leu Pro Phe Leu Gly Pro Leu Ala Ala Ile Ile Phe Leu Leu Leu Phe
    450                 455                 460

Gly Pro Cys Ile Phe Asn Phe Leu Val Lys Phe Val Ser Ser Arg Ile
465                 470                 475                 480

Glu Ala Val Lys Leu Gln Ile Val Leu Gln Met Glu Pro Gln Met Gln
                485                 490                 495

Ser Met Thr Lys Ile Tyr Arg Gly Pro Leu Asp Arg Pro Ala Arg Leu
            500                 505                 510

Cys Ser Asp Val Asn Asp Ile Glu Val Thr Pro Pro Glu Glu Ile Ser
        515                 520                 525

Thr Ala Gln Pro Leu Leu His Ser Asn Ser Val Gly Ser Ser
    530                 535                 540

<210>  10
<211>  11
<212>  PRT
<213>  protein ENV of the Human endogenous retrovirus-W

<400>  10

Leu Phe Asn Thr Thr Leu Thr Arg Leu His Glu
1               5                   10

<210>  11
<211>  7
<212>  PRT
<213>  protein ENV of the Human endogenous retrovirus-W

<400>  11

Leu Tyr Asn His Val Val Pro
1               5

<210>  12
<211>  336
<212>  DNA
<213>  VL

<400>  12
gatattgtga tgacgcaggc tgcattctcc aatccagtca ctcttggaac atcagcttcc      60

atctcctgca ggtctagtaa gagtctccta catagtaagg gcatcactta tttgtattgg     120

tttctgcaga agccaggcca gtctcctcag ctcctgattt atcagatgtc ccaccttgcc     180

tcaggagtcc cagacaggtt cagtagcagt gggtcaggaa ctgatttcac actgagaatc     240

agcagagtgg aggctgagga tgtgggtgtt tattactgtg ctcaaaatct agaacttccg     300

tggacgttcg gtggaggcac caagctggaa atcaaa                               336

<210>  13
<211>  348
<212>  DNA
<213>  artificial sequence

<220>
<223>  VH

<400>  13
gaggtccagc tgcagcagtc tgggactgta ctggcaaggc ctggggcttc cgtgaagatg      60

tcctgcaagg cttctggcta cagttttacc agctactgga tgcactggat aaaacagagg     120

cctggacagg gtctagaatg gattggtgct atttatccgg gaaaaagtga tactagctac     180

aaccagaagt ttaagggcaa ggccaagttg actgcggtca catccgccag cactgcctac     240

atggaactca ccagcctgac aaatgaggac tctgcggtct attattgtac aagaaccgtc 300

tatgctatgg actattgggg tcaaggaacc tcagtcaccg tctcctca 348

## Description

### FIELD OF THE INVENTION

The present invention relates to a newly identified group of patients previously diagnosed with a psychotic disease, in particular Schizophrenia or Bipolar disorder, in whom a high level of a pro-inflammatory cytokine in a body fluid sample, makes them eligible to a targeted treatment against HERV-W ENV.

### BACKGROUND OF THE INVENTION

Psychotic disorders are genetically- and environmentally-mediated mental illnesses that involve impairments in perception, mood, and social interactions (*1*). Until now, diagnoses of psychotic disorders are clinically-defined and are only treated by chemical compounds (26, 27, 28).

Most psychomimetic drugs are antagonists of the N-Methyl-D-aspartate receptors (NMDAR) such as phencyclidine (1-phenylcyclohexylpiperidin), also called PCP. Alterations of the NMDAR signaling have been associated with behavioral deficits in cognitive and sensorimotor gating test (33), which are hallmarks of psychosis models in rodents.

Although the pathogenic mechanisms underpinning these disorders are still unclear, it recently emerged that immune-related genes and mobile genetic elements constitute major actors in the etiopathogenetic chain (*1*-*3*). Mobile genetic elements, including human endogenous retroviruses (HERVs), are remnants of infections that took place several million years ago and embody around 8% of the human genome (4). Beside their initial classification as "junk" DNA, endogenous retroviruses capacity to control gene regulatory networks during human brain evolution and development (*5*-*8*), and their growing association to major neurological and psychiatric disorders (*9*, *10*) provide new conceptual framework to decrypt the interplay between immunological, genetic, and brain systems. HERVs are normally silenced by cell machineries, but they can be activated following immune challenges and under pathological conditions (*11*). For instance, patients diagnosed with schizophrenia and bipolar disorder have altered HERV gene expression, with variable transcriptional levels and protein detection (*1*, *12, 13).* Yet, whether the proteins expressed by HERV genes such as the envelope protein play an instrumental role in psychotic disorders and, most importantly, in which patients, still remains as unanswered questions with missing knowledge in the domain. This also precludes consistent indication for a targeted treatment in relevant patients when nosological definitions of psychiatric diseases are potentially resulting in heterogeneous populations without clearly identified etiology and with limited understanding of corresponding mechanisms of pathogenesis (*14*-*16*). To date, this absence of knowledge only allows targeting common downstream consequences that may modulate patients' symptomatology.

### SUMMARY OF THE INVENTION

For the first time, the inventors discovered that unexpectedly among the patients suffering from psychotic diseases, there was a subgroup of patients with a cytokine level significantly higher than that of the other patients, and that this subgroup corresponded to a subgroup of patients expressing HERV-W. They have shown the efficacy of an anti-HERV-W ENV antibody to treat this subgroup of psychotic patients having a high level of a cytokine in a body fluid sample, in particular in the serum.

The inventors have highlighted that HERV-W ENV altered NMDAR-mediated synaptic transmission in hippocampal networks, which appeared to be mediated via microglia and the release of pro-inflammatory cytokines such as IL-1β or IL-6. This involves innate immunity and pro-inflammatory cytokines in an HERV-W ENV-triggered specific pathogenic pathway. The glutamatergic GluN2B-containing NMDA receptor is normally present at the synapses, but an exposure to HERV-W ENV was shown to cause a surprising dispersal of GluN2B away from the synapses. Because of its biochemical nature, i.e. a protein macromolecule, HERV-W ENV was not expected to display molecular interactions leading to similar effects than those mediated by the small chemical compounds used to interfere with receptors of neuronal neurotransmitters but, surprisingly, the inventors have demonstrated its direct involvement in the psychotic phenotype. The delocalization of GluN2B-containing NMDA receptor renders them non-functional at synapse. The effect of the HERV-W ENV protein in animals reproduced features of psychotic disorders such as schizophrenia or bipolar disorder, and was accompanied by secretions of pro-inflammatory cytokines.

The Inventors showed that HERV-W ENV, detected in a biologically-defined relevant group of psychotic patients, is capable of generating a dysfunction in the NMDAR organization in conjunction with an induction of cytokine production, which affects long-term plasticity within glutamatergic synapses and produces behavioral deficits associated with psychosis.

Exploring the potential clinical and/or biological correlates of HERV-W ENV detection in patients diagnosed with Schizophrenia or with Bipolar disorder, the inventors evidenced different clusters of patients, across presently existing diagnoses. They identified a subgroup of patients with a significantly elevated level for a cytokine, in particular in the serum, and with detectable HERV-W ENV antigen and a subgroup without detectable HERV-W ENV antigen nor significant cytokine levels. These subgroups appear to represent biomarker-defined subgroups, only one of which is consistent with HERV-W ENV pathogenicity resulting in different clinical outcomes. Indeed, various clinical outcomes may occur with other pathogenic agents, e.g., Acquired Immune Deficiency Syndrome (AIDS) multifaceted diseases caused by HIV-1 retrovirus (*17*).

From these surprising results, the inventors have defined a new cluster of patients among the patients suffering from psychotic disease. The knowledge of this new indication has rendered possible the research of an appropriate treatment for this subgroup of patients. Unexpectedly, the inventors have discovered that the in vivo use of an antibody targeting HERV-W ENV could efficiently treat the neurobiological dysfunctions and psychotic symptoms in this subgroup of individuals.

Surprisingly, the use of an anti-HERV-W ENV antibody prevents the displacement from synapses and/or induces the relocation into synapses of GluN2B-containing NMDA glutamatergic receptor, and thus provides a beneficial effect on the pathogenic dysfunction of NMDA receptor synaptic neurotransmission. The inventors have therefore demonstrated that the use of an anti-HERV-W ENV antibody (also noted here anti-ENV or anti-ENV antibody) could specifically and efficiently reverse the pathogenic effects at the level of the GluN2B-containing NMDA receptor, when impacted through its biodistribution over neuronal synapses by HERV-W ENV.

In prior art, there was no indication of the identification of a subgroup of patients with HERV-W ENV expression among patients with psychotic disease, and that this subgroup also corresponded to a subgroup with a high level for a cytokine. Moreover, the presently and newly identified mode of action of HERV-W ENV on neuronal synaptic receptors indicates that this subgroup presents with a pathogenic protein (HERV-W ENV) involving a delocalization of GluN2B-containing NMDA receptor from the synapses. In addition, in prior art, there was no suggestion that HERV-W ENV protein has a fundamental role in the psychotic phenotype through its action on microglia-mediated specific cytokine release and NMDAR delocalization in synapses with specific involvement of GluN2B-containing NMDAR. Similarly and consistently, there was no suggestion that an antibody directed against HERV-W ENV could prevent and revert the alteration of the NMDAR signaling and, moreover, specifically on the GluN2B-containing NMDA receptor. Moreover, no identified nosological sub-groups could provide a defined indication for treating a relevant group of patients, i.e., schizophrenic or bipolar patients in which HERV-W ENV drives pathogenesis and not those from other sub-group(s) without HERV-W ENV and pro-inflammatory cytokine involvement. Thus, the efficiency of a treatment of psychotic disease with an anti-HERV-W ENV antibody along with a newly defined indication was totally unexpected, as there was no indication that this protein needed to be targeted in a biologically-defined sub-population of patients with diagnosis of psychotic disease and that the use of such antibody could thus target the delocalization of NMDAR.

Therefore,
in a **first aspect,** the invention relates to an anti-HERV-W ENV antibody which induces in neurons, the relocation of the GluN2B-containing NMDA receptor into the synapses, and in particular which binds to the conformational epitope of HERV-W ENV defined by the two distant linear sequences depicted in SEQ ID NO: 10 and in SEQ ID NO: 11, and more particularly which comprising each of the complementary-determining regions (CDRs) set forth in SEQ ID NO: 1, SEQ ID NO: 2, SEQ ID NO: 3, SEQ ID NO: 4, SEQ ID NO: 5 and SEQ ID NO: 6.
In a **second aspect,** the invention relates to a pharmaceutical composition comprising such an antibody and a pharmaceutically acceptable excipient.
In a **third aspect,** the invention relates to such an anti-HERV-W ENV antibody or such a pharmaceutical composition for use in the treatment of a subgroup of patients diagnosed with a psychotic disease and having a high level of a cytokine in a body fluid sample, in particular in the serum, said cytokine being in particular a pro-inflammatory cytokine, more particularly IL-6, IL-1β and /or TNF-α.
In a **fourth aspect,** the invention relates to a diagnostic method to identify if a patient diagnosed with a psychotic disease, belongs to a subgroup of patients suffering from psychotic disease as defined in the present disclosure, comprising:
   1) quantifying the level of a cytokine; and/or
   2) detecting the expression of HERV-W ENV
In a **fifth aspect,** the invention relates to a follow-up method of the efficacy of a treatment of a group of a patient suffering from a psychotic disease and having a high level of a cytokine, comprising the quantification of said cytokine and/or the detection of HERV-W ENV in a patient biological sample.

### DETAILED DESCRIPTION

### Definitions

As used herein, the terms **"human endogenous retrovirus"** and **"HERV",** refer to the human endogenous retroviruses that comprise the virus belonging to the type-W endogenous retrovirus family, usually named "HERV-W".

**"HERV-W"** is a family of human endogenous retroviruses that was unravelled in human genome from the initial discovery of "Multiple Sclerosis associated Retrovirus", MSRV, a human retrovirus first isolated from patients with multiple sclerosis. Therefore, when studies mention "LM7" (first isolate described from MS), "MS-retrovirus", "MSRV", "Syncytin", "HERV-W 7q", "ERVW-E1", "ERVW-E2", "HERV-W copies from X chromosome" or "HERV-W", they all designate HERV-W elements.
As used herein, the term **"MSRV"** refers to a specific endogenous retrovirus which is a member of the HERV-W family. In the context of the present invention, the expressions **"HERV-W"** and **"MSRV"** both designate HERV-W elements. Specifically, the expressions **"HERV-W Envelope protein", "HERV-W ENV", "MSRV-ENV"** and **"ENV"** altogether refer to the same envelope proteins. Typically, possible few variations in amino acid sequence do not prevent the binding of specific anti-ENV antibodies for therapeutic use in a HERV-W ENV associated psychotic disease.

As used herein, the term **"treating"** or **"treatment",** as used herein, means reversing, alleviating, inhibiting the progress of one or more symptoms of the disorder or condition to which such term applies.

As used herein, the term **"antibody"** refers to immunoglobulin molecules and immunologically active portions of immunoglobulin molecules, i.e., molecules that contain an antigen binding site that specifically binds an antigen. As such, the term "antibody" should be understood broadly and encompasses not only immunoglobulin molecules, but also antibody fragments, as well as derivatives of antibodies. In a particular embodiment, the antibody is an antibody directed against HERV-W ENV and inducing in neurons, the relocation of the GluN2B-containing NMDA receptor into the synapses. In a particular embodiment, the antibody comprises all the 6 CDRs as depicted in SEQ ID NO: 1 to 6.

As used herein, the expression "**fragment of antibody**" refers to a portion of such an antibody that mimics the hypervariable region, such as a CDR (CDR-L1, CDR-L2, CDR-L3, CDR-H1, CDR-H2, CDR-H3). The fragments of antibody according to the present invention retain the binding affinity and specificity of said antibody. Such fragments are functional equivalents of said antibody and they bind substantially to the same epitope as said antibody. Examples of fragments of antibody include but are not limited to heavy chain, light chain, VL, VH, Fv, Fab, Fab', F(ab)2, and F(ab')2.

As used herein, the expression "**derivative of antibody**" refers to a fragment of the antibody of the invention, preferably including the six CDRs of said antibody, fused to at least one sequence different from the natural sequence (e.g. a linker sequence of another species...), said derivative having binding affinity and specificity to HERV-W ENV comparable to that of the antibody of the invention. The derivatives according to the present invention retain the binding affinity and specificity of said antibody. Such derivatives are functional equivalents of said antibody and they bind at substantially the same epitope as said antibody. Examples of derivatives of antibody include, but are not limited to scFv, (scFv)2 and diabodies.

In natural antibodies, two heavy chains (HC) are linked to each other by disulfide bonds and each heavy chain is linked to a light chain (LC) by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains.

Typically, the light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine the binding site specific to the antigenic epitope. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody binding site and the antigenic epitope. Antibody binding sites are made up of residues that are primarily from the hypervariable or complementarity determining regions (CDRs). Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR-L1, CDR-L2, CDR-L3 and CDR-H1, CDR-H2, CDR-H3, respectively. An antigen-binding site, therefore, includes six CDRs, comprising the CDR set from each of a heavy and a light chain V region. Framework Regions (FRs) refer to amino acid sequences interposed between CDRs.

As used herein, the term "**chimeric antibody**" refers to an antibody which comprises a VH domain and a VL domain of an antibody from any species, preferably mouse, and a CH domain and a CL domain of a human antibody.

According to the invention, the term "**humanized antibody**" refers to an antibody having variable region framework and constant regions from a human antibody but retains the CDRs of an antibody from any species, preferably mouse.

The term "Fab" denotes an antibody fragment having a molecular weight of about 50,000 and antigen binding activity, in which about a half of the N-terminal side of H chain and the entire L chain, among fragments obtained by treating IgG with a protease, papain, are bound together through a disulfide bond.

As used herein, the term **"F(ab')2"** refers to an antibody fragment having a molecular weight of about 100,000 and antigen binding activity, which is slightly larger than the Fab bound via a disulfide bond of the hinge region, among fragments obtained by treating IgG with a protease, pepsin.

As used herein, the term **"Fab'** " refers to an antibody fragment having a molecular weight of about 50,000 and antigen binding activity, which is obtained by cutting a disulfide bond of the hinge region of the F(ab')2.

The expressions "**A single chain Fv**" or "**scFv**"" refer to a polypeptide which is a covalently linked VH::VL heterodimer, and usually expressed from a gene fusion including VH and VL encoding genes linked by a peptide-encoding linker. "**dsFv**" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "**diabodies**" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

As used herein, the expression **"antibody of the invention"** refers to an antibody directed against, i.e. that specifically binds to HERV-W Envelope protein (HERV-W ENV), preferably against HERV-W ENV protein of the type-W human endogenous retrovirus family (HERV-W), more preferably against the envelope protein of MSRV, more preferably against the extracellular domain the envelope protein of MSRV. In one embodiment, the antibody according to the invention is an antibody directed against HERV-W ENV and inducing in neurons, the relocation of the GluN2B-containing NMDA receptor, into the synapses. The antibody of the invention typically binds to the conformational epitope.

In particular, the antibody of the invention binds to the conformational epitope defined by the two distant linear sequences depicted in SEQ ID NO: 10 and in SEQ ID NO: 11.

Preferably, the antibody of the invention comprises all the 6 CDRs as depicted in SEQ ID NO: 1 to 6.

Within the meaning of the invention the expressions "anti-ENV antibody", "anti-HERV-W ENV antibody", "antibody directed against HERV-W ENV" and "antibody targeting HERV-W ENV" are used indifferently and are equivalent.

As used herein, the term **"biological sample"** as used herein refers to any biological sample obtained for the purpose of evaluation *in vitro.* In the present invention, the sample or patient sample may comprise any body fluid or disease-specific tissue and lesions. Examples of body fluid include blood, nipple aspirate fluid, urine, saliva, synovial fluid and cerebrospinal fluid (CSF). In particular, a blood sample can be serum or plasma.

### Antibody of the invention

The present invention relates to an antibody directed against HERV-W ENV and inducing in neurons, the relocation of the NMDAR, in particular of the NMDA receptor containing the GluN2B subunit, into the synapses.
There are two main subtypes of the NMDA receptor (also noted NMDAR) in neurons. The NMDAR which contains the GluN2A subunit, called GluN2A-containing NMDA receptor (GluN2A-NMDAR), and the NMDAR which contains the GluN2B subunit, called GluN2B-containing NMDA receptor (GluN2B-NMDAR).

The present invention also relates to an anti-HERV-W ENV antibody binding to a conformational epitope of HERV-W ENV.

The term **"conformational"** opposes to the term "linear". In the present invention, it means that the epitope is seen as two separated and distant linear amino acid sequences that are significantly detected by the same monoclonal antibody in classical epitope mapping protocols with overlapping peptides covering the primary amino acid sequence of the protein. The conformational epitope is formed when the protein is folded into a specific three-dimensional shape that presents these two distant amino acid sequences as contiguous ones, therefore targeted as a single unique epitope consisting in the joint sequences.

Preferably, the anti-HERV-W ENV antibody binds to the conformational epitope of HERV-W ENV defined by the two distant linear sequences depicted in SEQ ID NO:10 and in SEQ ID NO:11.

It is well-known by a person ordinary skilled in the art how to generate antibodies binding to a specific epitope defined by its aminoacid sequence (29)

The present invention further relates to an antibody comprising each of the complementary-determining regions (CDRs) set forth in SEQ ID NO: 1, SEQ ID NO: 2, SEQ ID NO: 3 SEQ ID NO: 4, SEQ ID NO: 5 and SEQ ID NO: 6.

The present invention also relates to an anti-HERV-W ENV antibody which induces in neurons, the relocation of the GluN2B-containing NMDA receptor into the synapses, and which comprises each of the complementary-determining regions (CDRs) set forth in SEQ ID NO:1, SEQ ID NO: 2, SEQ ID NO: 3 SEQ ID NO: 4, SEQ ID NO: 5 and SEQ ID NO: 6.

The present invention also relates to an anti-HERV-W ENV antibody which induces in neurons, the relocation of the GluN2B-containing NMDA receptor into the synapses, and which binds to the conformational epitope of HERV-W ENV defined by the two distant linear sequences depicted in SEQ ID NO: 10 and in SEQ ID NO: 11.

The present invention also relates to an anti-HERV-W ENV antibody binding to the conformational epitope of HERV-W ENV defined by the two distant linear sequences depicted in SEQ ID NO: 10 and in SEQ ID NO: 11, and comprising each of the complementary-determining regions (CDRs) set forth in SEQ ID NO: 1, SEQ ID NO: 2, SEQ ID NO: 3, SEQ ID NO: 4, SEQ ID NO: 5 and SEQ ID NO: 6.

The present invention also relates to an anti-HERV-W ENV antibody binding to the conformational epitope of HERV-W ENV defined by the two distant linear sequences depicted in SEQ ID NO: 10 and in SEQ ID NO: 11, and comprising each of the complementary-determining regions (CDRs) set forth in SEQ ID NO: 1, SEQ ID NO: 2, SEQ ID NO: 3, SEQ ID NO: 4, SEQ ID NO: 5 and SEQ ID NO: 6, and which induces in neurons, the relocation of the GluN2B-containing NMDA receptor into the synapses.

In one embodiment, the antibody of the invention comprises:
- a light chain wherein the variable domain (VL) comprises each of the 3 CDRs as depicted in SEQ ID NO: 1 for CDR-L1, SEQ ID NO: 2 for CDR-L2 and SEQ ID NO: 3 for CDR-L3; and
- a heavy chain wherein the variable domain (VH) comprises each of the 3CDRs as depicted in SEQ ID NO: 4 for CDR-H1, SEQ ID NO: 5 for CDR-H2 and SEQ ID NO: 6 for CDR-H3.

The above mentioned complementarity determining regions (CDRs) are disclosed in Table 1:

**Table 1: CDR domains of an antibody according to the invention**

| **Domains** | **SEQ ID NO:** | **Sequence** |
|---|---|---|
| CDR-L1 | 1 | KSLLHSKGITY |
| CDR-L2 | 2 | QMS |
| CDR-L3 | 3 | AQNLELPWT |
| CDR-H1 | 4 | GYSFTSYW |
| CDR-H2 | 5 | IYPGKSDTS |
| CDR-H3 | 6 | TRTVYAMDY |

In one embodiment, the antibody of the invention comprises:
- a light chain variable region (VL) as depicted in SEQ ID NO: 7; and
- a heavy chain variable region (VH) as depicted in SEQ ID NO: 8.

The above mentioned light and heavy variable regions are disclosed in Table 2:

**Table 2 : Light and heavy variable regions of an antibody according to the invention**

| **Domains** | **SEQ ID NO:** | **Sequence** |
|---|---|---|
| VL | 7 | DIVMTQAAFSNPVTLGTSASISCRSSKSLLHSKGITYLYWFL QKPGQSPQLLIYQMSHLASGVPDRFSSSGSGTDFTLRISRVE AEDVGVYYCAQNLELPWTFGGGTKLEIK |
| VH | 8 | EVQLQQSGTVLARPGASVKMSCKASGYSFTSYWMHWIKQ RPGQGLEWIGAIYPGKSDTSYNQKFKGKAKLTAVTSASTA YMELTSLTNEDSAVYYCTRTVYA MDYWGQGTSVTVSS |

In one embodiment, the present invention relates to a fragment or a derivative of the antibody described above, selected from the group consisting of a Fv, Fab, F(ab')2, Fab', dsFv, scFv, sc(Fv)2, a diabody.

In a preferred embodiment, the antibody of the invention is a monoclonal antibody. Monoclonal antibodies of the invention are monovalent, bivalent, multivalent, monospecific or bispecific. In another embodiment, the antibody directed against HERV-W ENV is a binding fragment or a conjugate. For examples antibodies of the invention may be conjugated to a growth inhibitory agent, cytotoxic agent, or a prodrug-activating enzyme.

Another type of amino acid modification of the antibody of the invention may be useful for altering the original glycosylation pattern of the antibody. By "altering" is meant deleting one or more carbohydrate moieties found in the antibody, and/or adding one or more glycosylation sites that are not present in the antibody. Glycosylation of antibodies is typically N-linked. "N-linked" refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. Addition of glycosylation sites to the antibody is conveniently accomplished by altering the amino acid sequence such that it contains one or more of the above-described tripeptide sequences (for N-linked glycosylation sites).

In a particular embodiment, the anti-HERV-W ENV antibody according to the invention is a monoclonal antibody, a chimeric monoclonal antibody, or a humanized monoclonal antibody.

Preferably, said antibody anti-HERV-W ENV is an IgG, in particular an IgG1 or an IgG4, preferably an IgG4.

More preferably, the antibody of the invention is a humanized monoclonal antibody, more preferably a humanized IgG4 monoclonal antibody or a humanized IgG1 monoclonal antibody.

Said humanized antibody may be produced by obtaining nucleic acid sequences encoding for CDRs domain by inserting them into an expression vector for animal cell having genes encoding a heavy chain constant region identical to that of a human antibody; and a light chain constant region identical to that of a human antibody, and expressing the expression vector by introducing it into an animal cell. The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, a tandem type of the humanized antibody expression vector is more preferable. Examples of the tandem type humanized antibody expression vector include pKANTEX93, pEE18 and the like. Methods for producing humanized antibodies based on conventional recombinant DNA and gene transfection techniques are well known in the art. Antibodies can be humanized using a variety of techniques known in the art including, for example, CDR-grafting, veneering or resurfacing, and chain shuffling. The general recombinant DNA technology for preparation of such antibodies is also known.

Thus, an embodiment of the invention relates to a monoclonal humanized antibody comprising:
- a light chain wherein the variable domain comprises each of the 3 CDRs as depicted in SEQ ID NO: 1 for CDR-L1, SEQ ID NO: 2 for CDR-L2 and SEQ ID NO: 3 for CDR-L3; and
- a heavy chain wherein the variable domain comprises each of the 3CDRs as depicted in SEQ ID NO: 4 for CDR-H1, SEQ ID NO: 5 for CDR-H2 and SEQ ID NO: 6 for CDR-H3.

### Antibody for therapeutic use and for use in the treatment of a psychotic disease

The present invention also relates to an anti-HERV-W ENV antibody described above for a therapeutic use or for use as a medicament.

In one embodiment, the present invention relates to an antibody directed against HERV-W ENV and inducing in neurons, the relocation of the GluN2B-containing NMDA receptor, into the synapses, for a therapeutic use for use as a medicament.

In one embodiment, the present invention relates to an antibody directed against HERV-W ENV for a therapeutic use for use as a medicament, wherein said antibody binds to the conformational epitope of HERV-W ENV defined by the two distant linear sequences depicted in SEQ ID NO: 10 and in SEQ ID NO: 11.
In one embodiment, the present invention relates to an antibody directed against HERV-W ENV for a therapeutic use for use as a medicament, wherein said antibody comprises each of the complementary-determining regions (CDRs) set forth in SEQ ID NO: 1, SEQ ID NO: 2, SEQ ID NO: 3, SEQ ID NO: 4, SEQ ID NO: 5 and SEQ ID NO: 6.

These three embodiments may be combined with each other.

The present invention also relates to an anti-HERV-W ENV antibody described above for treating a subgroup of patients diagnosed with a psychotic disease and having a high level of a cytokine in a body fluid sample, in particular in a blood sample, more particularly in the serum or the plasma.

More particularly, the present invention relates to an antibody directed against HERV-W envelope protein (ENV) for use in the treatment of a group of patients suffering from a psychotic disease and having a high level for a cytokine in a body fluid sample, in particular in the blood, more particularly in the serum or the plasma.

A "high level for a cytokine" or "high level of a cytokine" as used herein means that the level of said cytokine within the group of patients is significantly higher than those of healthy persons. As an example, a high level of a cytokine is a level of a cytokine higher than 0.5 pg/ml for IL-6 in the serum, higher than 0.05 pg/ml for IL-1β in the serum, higher than 1.25 pg/ml for TNF-α in the serum. Of note, the thresholds of cytokines quoted in the present disclosure vary with the kits, antibodies, techniques, protocols, devices and platforms used for their dosages and their sensitivity (proportion of true-positives) and their specificity (proportion of true-negatives). Presented values are only indicative and one should adapt them according to manufacturers' instructions or assess them with a given platform. In other words, it means that the level of said cytokine in the group of patients is significantly above normal level of said cytokine. This normal value is given by kit suppliers and/or can be determined from a panel of healthy individuals. A person skilled in the art perfectly knows how to measure and how to determine such a threshold or normal level for a given cytokine according to the different techniques, protocols and devices available on the market.

This definition applies to the high levels of the specific cytokines (such as IL-6, IL-1β and TNF-α) quoted in the present disclosure.

Typically, the level of a cytokine is measured in a body fluid sample, in particular in a blood sample, more particularly in the serum or in the plasma.

In the present disclosure, the expressions "group of patients", "subgroup of patients" and "cluster of patients" are used indifferently and are equivalent.

A patient suffering from a psychotic disease is a patient with psychotic symptoms or diagnosed with a psychotic disease.

In a particular embodiment, the present invention relates to an anti-HERV-W ENV antibody for use in the treatment of a group of patients suffering from a psychotic disease and having a high level for a cytokine in a body fluid sample, wherein said anti-HERV-W ENV antibody induces the relocation of the NMDA receptor in neurons, in particular of the GluN2B-containing NMDA receptor, into the synapses.

This means that said anti-HERV-W ENV antibody is able to reverse the dispersal of NMDAR from neuronal synapses in which its can have again a normal functional activity. This relates particularly to the glutamatergic GluN2B-containing NMDA receptor. In fact, as demonstrated in Example 2, HERV-W ENV causes the loss of functional activity of the NMDAR by inducing its delocalization from the physiologically relevant synaptic area. The particular dispersal of the GluN2B-containing NMDA receptor away from the synapses to the postsynaptic compartment (i.e., in neuronal membrane regions out of the synapses), leads to characteristic symptoms of psychotic disorders.

Typically, the Example 2 teaches how to identify the activity of an antibody anti-HERV-W on the relocation of the GluN2B-containing NMDA receptor from the postsynaptic compartment to the synapses, in hippocampal neurons. Animal hippocampi are widely used in neurology, as they constitute an animal cell model for the study of psychotic disorders and as they correspond to an easily accessible brain area.

As neurons in hippocampus are equivalent to neurons found in other brain areas for their global synaptic functions and for their need for synaptic localization of receptors to neurotransmitters, observations made on hippocampal neurons are extrapolated to neurons in other brain areas. Thus, although the brain areas involved in different psychotic pathologies may vary, the present observations made on neurons of the hippocampus are representative of neurons located in other brain areas.

In a particular embodiment, the present invention relates to an anti-HERV-W ENV antibody for use in the treatment of a group of patients suffering from a psychotic disease and having a high level for a cytokine in a body fluid sample, wherein said antibody binds to a conformational epitope of HERV-W ENV. More particularly, said conformational epitope is defined by the two distant linear sequences depicted in SEQ ID NO: 10 and in SEQ ID NO: 11.

In a particular embodiment, the present invention relates to an anti-HERV-W ENV antibody directed against HERV-W ENV comprising each of the 6 CDRs as depicted in SEQ ID NO: 1, SEQ ID NO: 2, SEQ ID NO: 3, SEQ ID NO: 4, SEQ ID NO: 5 and SEQ ID NO: 6, for use in the treatment of a group of patients suffering from a psychotic disease and having a high level for a cytokine in a body fluid sample.

In a particular embodiment, the present invention relates to an antibody directed against HERV-W ENV which induces in neurons, the relocation of the NMDA receptor, in particular of the NMDA receptor containing the GluN2B subunit, into the synapses, and which comprises each of the 6 CDRs as depicted in SEQ ID NO: 1, SEQ ID NO: 2, SEQ ID NO: 3, SEQ ID NO: 4, SEQ ID NO: 5 and SEQ ID NO: 6, for use in the treatment of a group of patients suffering from a psychotic disease and having a high level for a cytokine in a body fluid sample.

In a particular embodiment, the present invention relates to an antibody directed against HERV-W ENV which induces in neurons, the relocation of the NMDA receptor, in particular of the NMDA receptor containing the GluN2B subunit, into the synapses, which specifically binds to the conformational epitope defined by the two distant linear sequences depicted in SEQ ID NO: 10 and in SEQ ID NO: 11 and which comprises each of the 6 CDRs as depicted in SEQ ID NO: 1, SEQ ID NO: 2, SEQ ID NO: 3, SEQ ID NO: 4, SEQ ID NO: 5 and SEQ ID NO: 6, for use in the treatment of a group of patients suffering from a psychotic disease and having a high level for a cytokine in a body fluid sample.

In particular, said psychotic disease is selected from the group consisting of schizophrenia, bipolar disorder, schizoaffective psychosis and schizophreniform disorder. These pathologies are defined according to the DSM IV or V classification or to an appropriate classification for psychotic diseases (*25*-*28*). The diagnosis of psychotic disease, and more particularly of this list of psychotic diseases, is well known from the practitioners. More preferably, said psychotic disease is schizophrenia or bipolar disorder.

In particular, said cytokine is a pro-inflammatory cytokine. More particularly, said pro-inflammatory cytokine is selected in the group consisting of IL-6, IL-1β and TNF-α.

In a particular embodiment, the present invention relates to an antibody directed against HERV-W envelope protein (ENV) for use in the treatment of a group of patients suffering from a psychotic disease and having a high level for a cytokine in a body fluid sample, in particular in a blood sample, more particularly in the serum, said cytokine being in particular a pro-inflammatory cytokine, more particularly IL-6, IL-1β and /or TNF-α.

In a particular embodiment, said psychotic disease is Schizophrenia and said cytokine with a high level in a body fluid sample is preferably IL-6.

In a particular embodiment, said psychotic disease is Bipolar disorder and said cytokine with a high level in a body fluid sample is preferably IL-1β. In a more particular embodiment, said group of patients suffering from Bipolar disorder and having a high level for IL-1β in a body fluid sample, further have a high level of TNF-α in a body fluid sample.

In a particular embodiment, the HERV-W ENV expression has been detected in the patients of said group. Since the subgroup of patients presenting a high level of cytokines corresponds to the subgroup of patients expressing HERV-W ENV, thus the detection of the HERV-W ENV, is not mandatory and may be optional. In particular, the HERV-W ENV is detected in a biological sample. More particularly, the HERV-W ENV is detected in a blood sample, more particularly in the serum or in the plasma. HEVR-W ENV is detected with an anti-HERV-W ENV antibody such as an anti-HERV-W ENV antibody according to the present disclosure.

The expression of HERV-W ENV can be characterized by the detection of HERV-W DNA, RNA, antigen or protein, following the below-described conditions.

HERV-W family was discovered after MSRV, a human retrovirus first isolated from patients with multiple Sclerosis (*18*-*20*)*.* Identification of the expression of HERV-W ENV is well-known by a person of ordinary skill in the art. Associated diseases or syndromes are defined by the presence in corresponding patients either (i) of specific HERV-W RNA or antigens, preferably detected in body fluids (blood, cerebrospinal fluid, urine...), either (ii) of elevated DNA or RNA copy number in cells or tissues from organs with lesions or dysfunctions, either (iii) of specific MSRV proteins or antigens in cells or tissues involved in the process of the disease or of the clinical syndrome, or (iv) of HERV-W proteins or antigens in body fluids of individuals with the disease or expressing the clinical syndrome (see international application WO2019201908A1 - Method for the detection of the soluble hydrophilic oligomeric form of HERV-W envelope protein). Examples of such detection assays with various technical conditions and targets have been described (*21*-*23*)*.* All these conditions of detection exclude the detection of the physiological HERV-W copy, Syncytin-1 when expressed in normal and physiological conditions of its expression, i.e. during pregnancy (24).
In such an embodiment, the psychotic disease may be also called "HERV-W ENV associated psychotic disease". Among the patients suffering from a psychotic disease, said group of patients is then a group of patients suffering from a psychotic disease, having a high level for a cytokine in a body fluid sample, in particular IL-6, IL-1β and/or TNF-α, and wherein HERV-W ENV has been detected, for example by detecting HERV-W DNA, RNA, antigen or protein, following the above-described conditions. In a particular embodiment, the present invention applies to the group of patients suffering from a psychotic disease, in whom the detection of the HERV-W ENV is characterized by the detection of HERV-W DNA, RNA, antigen or protein and/or having a high level for a cytokine in a body fluid sample.

In a particular embodiment, said anti-HERV-W antibody described above for use in the treatment of a group of patients suffering from a psychotic disease and having a high level for a cytokine in a body fluid sample, is a monoclonal antibody or a humanized monoclonal antibody. More particularly, said antibody is an IgG such as an IgG1 or an IgG4. More particularly, it is a humanized IgG4 monoclonal antibody or an IgG1 monoclonal antibody.

In a particular embodiment said anti-HERV-W antibody described above for use in the treatment of a group of patients suffering from a psychotic disease and having a high level for a cytokine in a body fluid sample, comprises:
- a light chain wherein the variable domain (VL) comprises each of the 3 CDRs as depicted in SEQ ID NO: 1 for CDR-L1, SEQ ID NO: 2 for CDR-L2 and SEQ ID NO: 3 for CDR-L3; and
- a heavy chain wherein the variable domain (VH) comprises each of the 3CDRs as depicted in SEQ ID NO: 4 for CDR-H1, SEQ ID NO: 5 for CDR-H2 and SEQ ID NO: 6 for CDR-H3.

In a particular embodiment, said anti-HERV-W antibody for use in the treatment of a group of patients suffering from a psychotic disease and having a high level for a cytokine in a body fluid sample, is an antibody comprising:
- a light chain variable region (VL) as depicted in SEQ ID NO: 7; and
- a heavy chain variable region (VH) as depicted in SEQ ID NO: 8.

It may also be a fragment or a derivative of an anti-HERV-W antibody described above, selected from the group consisting of a Fv, Fab, F(ab')2, Fab', dsFv, scFv, sc(Fv)2, a diabody.

### Pharmaceutical composition

A further object of the invention relates to a pharmaceutical composition comprising an antibody directed against HERV-W ENV as described above, or a fragment or a derivative thereof, and a pharmaceutically acceptable excipient. In particular, the pharmaceutical composition comprises an effective amount of said antibody.

The present invention also relates to such a pharmaceutical composition for use in the treatment of a group of patients suffering from a psychotic disease and having a high level for a cytokine in a body fluid sample, as previously described.

Any therapeutic agent of the invention as above described may be combined with pharmaceutically acceptable excipients, and optionally sustained-release matrices, such as biodegradable polymers, to form therapeutic compositions.

**"Pharmaceutically"** or **"pharmaceutically acceptable"** refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.
The pharmaceutical compositions of the invention can be formulated for a topical, oral, intranasal, intraocular, intravenous, intrathecal (directly in the cerebrospinal fluid), intramuscular or subcutaneous administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.
To prepare pharmaceutical compositions, an effective amount of the antibody directed against HERV-W ENV, or a fragment or a derivative thereof, may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol and in oils. Under ordinary conditions of storage and use, these preparations contain a preservative to prevent the growth of microorganisms.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

Preferably, the antibody directed against HERV-W ENV of the invention can be formulated into a buffer in which it was solubilized, stored and injected to patients. Preferably, said buffer comprises 20mM histidine, 5% sucrose, and 0.01% polysorbate 20.

For parenteral administration in an aqueous solution, for example, the solution may be suitably buffered and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the patient being treated. The person responsible for administration will, in any event, determine the appropriate dose for the individual patient.

In preferred embodiments, the antibody of the invention is formulated to be administered by intravenous or by intrathecal injection.

### Diagnostic method to identify a subgroup of patients suffering from psychotic disease

The present application also relates to a diagnostic method to identify if a patient diagnosed with a psychotic disease, belongs to a subgroup of patients suffering from psychotic disease as defined in the present disclosure, comprising:
1) quantifying the level of a cytokine; and/or
2) detecting the expression of HERV-W ENV.

In particular said cytokine is a pro-inflammatory cytokine, more particularly IL-6, IL-1β and/or TNF-α. The psychotic disease is selected from the group consisting of schizophrenia, bipolar disorder, schizoaffective psychosis and schizophreniform disorder. For example, when the patient is diagnosed with schizophrenia, the level of IL-6 is preferably quantified. For example, when the patient is diagnosed with bipolar disorder, the level of IL-1β is preferably quantified. In case of bipolar disorder, in addition the level of TNF-α may also be quantified. Typically, the level of cytokine is quantified in a body fluid sample, in particular in a blood sample, more particularly in the serum or in the plasma.

If the patient presents a high level of said cytokine in the body fluid sample, it means that said patient belongs to a subgroup of patients suffering from psychostic disease and characterized by a high level of a cytokine in a body fluid sample, and also characterised by the expression or the detection of the HERV-W ENV.
The definition of "high level of a cytokine" given above also applies here.
To detect the expression of HERV-W ENV, an anti-HERV-W ENV antibody such as one disclosed in the present application can be used. In the present disclosure, HERV-W ENV is detected in a biological sample, in particular in a body fluid sample such as a blood sample, more particularly in the serum or in the plasma.
Since the subgroup of patients presenting a high level of a cytokine corresponds to a subgroup of patients expressing HERV-W ENV, i. e. a subgroup of patients wherein HERV-W ENV is detected, identifying this subgroup of patients suffering from a psychotic disease therefore does not necessitate both quantifying the level of a cytokine and detecting HERV-W ENV. Thus, analysing only one of these parameters is sufficient.

### Therapeutic method and monitoring method according to the invention

In another aspect, the invention also relates to a method for treating a group of patients suffering from a psychotic disease and having a high level of a cytokine in a body fluid sample, comprising administering to said patients an effective amount of an anti-HERV-W ENV antibody as described in the present disclosure.

In another aspect, the invention also relates to the use of an anti-HERV-W ENV antibody as described in the present disclosure for the manufacture of a medicament for treating a group of patients suffering from a psychotic disease and having a high level of a cytokine in a body fluid sample.

All the embodiments described above apply to these other aspects of the invention.

The invention also relates to a method of treatment of a patient suffering from a psychotic disease, comprising the steps of:
1) quantifying the level of a cytokine; and/or
2) detecting the expression of HERV-W ENV.

In particular said cytokine is a pro-inflammatory cytokine, more particularly IL-6, IL-1β and/or TNF-α. In particular, the level of said cytokine is quantified in a blood sample, more particularly in the serum or in the plasma. To quantify the level of the cytokine, one or more specific biomarkers of said cytokine can be used. To detect the expression of HERV-W ENV an anti-HERV-W ENV antibody can be used such as one disclosed in the present application can be used. HERV-W ENV is detected in a biological sample, in particular in a body fluid sample such as a blood sample, more particularly in the serum or in the plasma.
If said step 1) shows a high level of a cytokine in a body fluid sample and/or if said step 2) shows the detection of HERV-W ENV, then the method of the invention comprises in addition a step of providing an antibody as described in the present disclosure to said patient. It should be noted that step 2) may be optional.
To determine a "high level of a cytokine", the definition of "high level of a cytokine" given above also applies here.
A follow-up may then be established using specific biomarkers of the cytokine(s) relevant for the patient or an anti-HERV-W ENV antibody such as one disclosed in the present application.

In a particular embodiment, the invention also relates to a method of treatment of a patient suffering from schizophrenia, comprising the steps of:
1) quantifying the level of IL-6; and/or
2) detecting the expression of HERV-W ENV.

If said step 1) shows a high level of IL-6 and/or if said step 2) shows the detection of HERV-W ENV, then the method of the invention comprises in addition a step of providing the antibody of the invention to said patient.

In a particular embodiment, the invention also relates to a method of treatment of a patient suffering from bipolar disorder, comprising the steps of:
1) quantifying the level of IL-1β; and/or
2) detecting the expression of HERV-W ENV;
3) optionally, quantifying the level of TNF-α.

If said step 1) shows a high level of IL-1β and/or if said step 2) shows the detection of HERV-W ENV, and optionally step 3) shows a high level of TNF-α, then the method of the invention comprises in addition a step of providing the antibody of the invention to said patient.

The invention also relates to a method for monitoring the response to a treatment of a patient suffering from a psychotic disease and having a high level of a cytokine in a body fluid sample, said method comprising the following steps:
1) quantifying the level of a cytokine;
2) detecting the HERV-W ENV protein.

In particular said cytokine is a pro-inflammatory cytokine, more particularly IL-6, IL-1β and/or TNF-α. In particular, the level of cytokine is quantified in a blood sample, more particularly in the serum or in the plasma. To quantify the level of the cytokine, one or more specific biomarkers of said cytokine can be used. To detect the expression of HERV-W ENV, an anti-HERV-W ENV antibody can be used such as one disclosed in the present application can be used. HERV-W ENV is detected in a biological sample, in particular in a body fluid sample such as a blood sample, more particularly in the serum or in the plasma. For example, if the psychotic disease is schizophrenia, said cytokine is preferably IL-6. For example, if said psychotic disease is bipolar disorder, said cytokine is preferably IL-1β, and optionally in addition preferably TNF-α.

The decrease of the cytokine level and/or the decrease of HERV-W ENV protein in the patient are indicators of the efficacy of the treatment. In addition, a behavioral study and/or clinical rating scales of the patients may be carried out to analyse an improvement of the general status of the patients and thus to confirm the efficacy of the treatment and/or to adapt its dosage to the patient.

Typically, in the present application, the step of detection and/or quantification may be performed according to the routine techniques, well known of the person skilled in the art. Typically, said step comprises contacting a biological sample such as a body fluid sample of the patient with selective reagents such as probes, primers, ligands or antibodies, and thereby detecting the presence of nucleic acids or proteins of interest originally in the sample.

### FIGURES LEGENDS

In all the legends and the figures, "Cont." means Control.

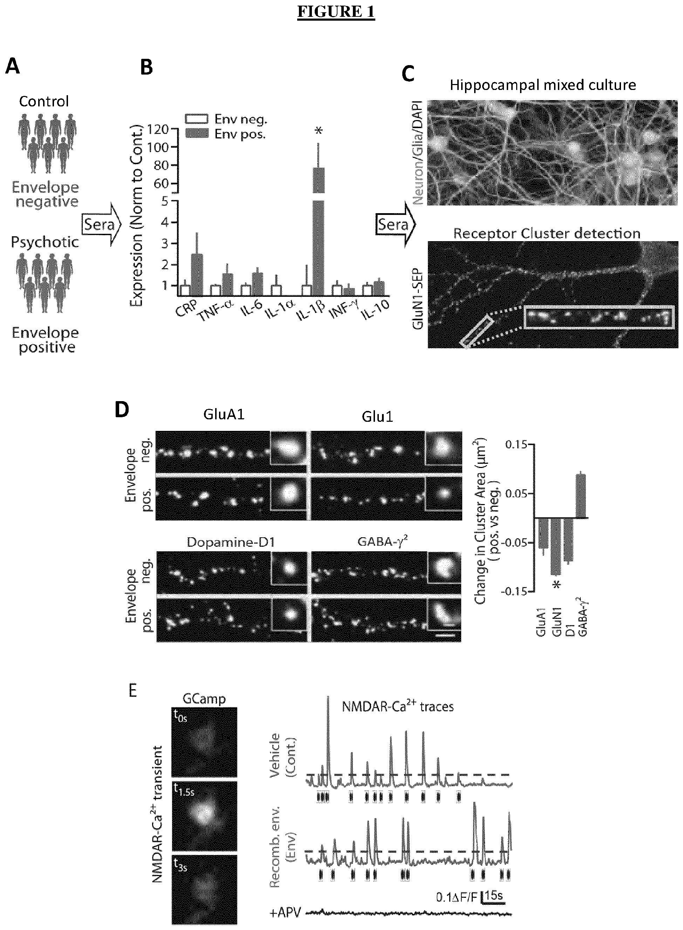

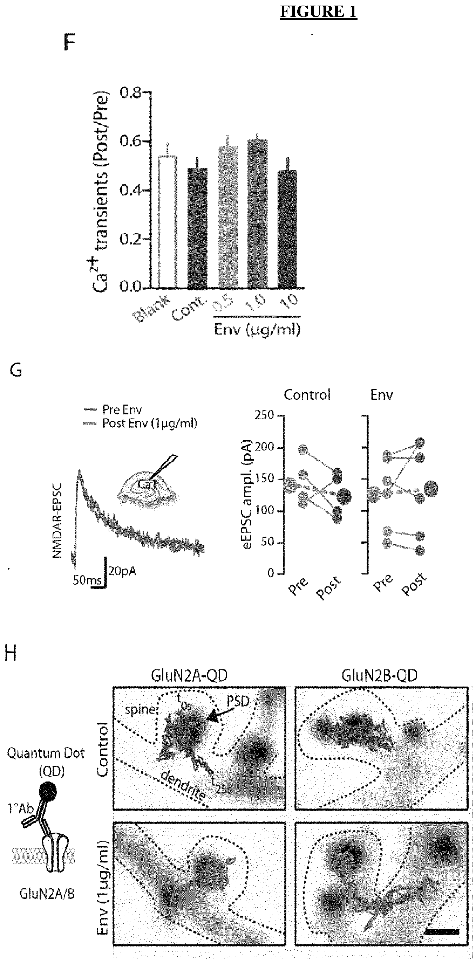

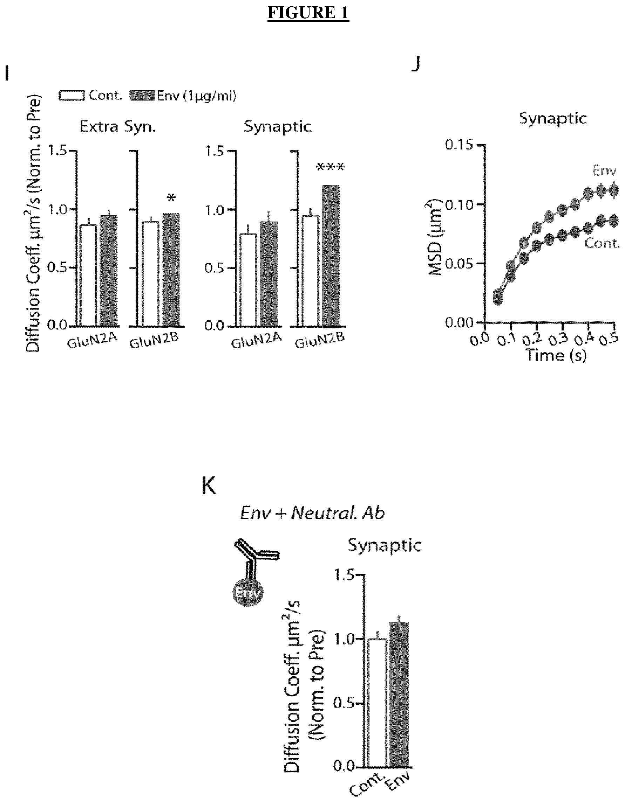

**FIGURE 1****: Sera from patients diagnosed with Schizophrenia or bipolar disorder with detectable HERV-W ENV (ENV) protein (positive antigenemia) induce neurotransmitter disturbance (NMDAR specific reduction and synaptic dispersion), with same characteristics evidenced by recombinant ENV, which can be reversed or prevented by anti-HERV-W ENV antibody.**
   **(A** and **B)** Increase of interleukin-1β levels in envelope-positive (pos., n = 6) serum samples from psychotic patients (neg., n = 5). **P* = 0.043, Student's t-test. **(C)** Representative image of cultured network and transfected neuron after 15 min serum-sample incubation. Scale bars, top/bottom = 30/10µm. **(D)** ENV-pos. serum-samples specifically decreases GluN1 cluster areas (inset). GluA1-SEP (n = 57/43 neurons neg./pos.), GluN1-SEP (n = 56/61), Dopaminel-CFP (n = 54/61) and GABAγ₂-SEP (n = 59/68). Scale bars = 1µm and inset 0.3µm. **P* = 0.048, Student's t-test. **(E** and **F)** NMDAR-mediated Ca²⁺ traces recorded in spines, dots represent detected transients, after 5 min recombinant envelope protein (ENV) or vehicle (Cont.) exposure. D-AP5 (50 µM) block all transients. Scale bar = 1µm. (**F**) Ca²⁺-transients ratio for Blank (n = 47/5 spines/neurons), Cont. (n = 92/7), ENV: 0.5µg/ml (n = 87/6), 1.0µg/ml (n = 114/9) and 10µg/ml (n = 18/1). **(G)** Representative NMDA traces and mean peak amplitude before (pre) and 5min post Cont. (n = 4 neurons) or ENV (1µg/ml, n = 6) application. **(H)** Representative trajectories of GluN2A- and GluN2B-NMDAR-QD complexes at post synaptic densities (PSD) 5min after Cont. or ENV (1µg/ml) exposure. Scale bar = 1µm. (**I**) ENV induce specific increase in GluN2B-containing NMDAR-receptors surface diffusion predominantly in synaptic areas. Data are normalized to pre-exposure for individual neurons. GluN2A: *Extra Syn*.: Cont. n = (300/4 trajectories/neurons) ENV (n = 445/5), *Synaptic*: Cont. (n = 176/4), ENV (n = 200/5). GluN2B: *Extra Syn*.: Cont. (n = 819/10), ENV (n = 727/10), *Synaptic*: Cont. (n = 670/10), ENV (n = 792/10). **P* = 0.0319 and ****P* < 0.0001, Mann-Whitney tests. **(J)** Mean square displacements (MSDs) for synaptic data in (I). (**K**) Co-application of anti-HERV-W ENV monoclonal antibody abolishes the ENV effect, Cont. (n = 407/7), ENV (n = 484/6). Bars and dots are mean ± SEM.

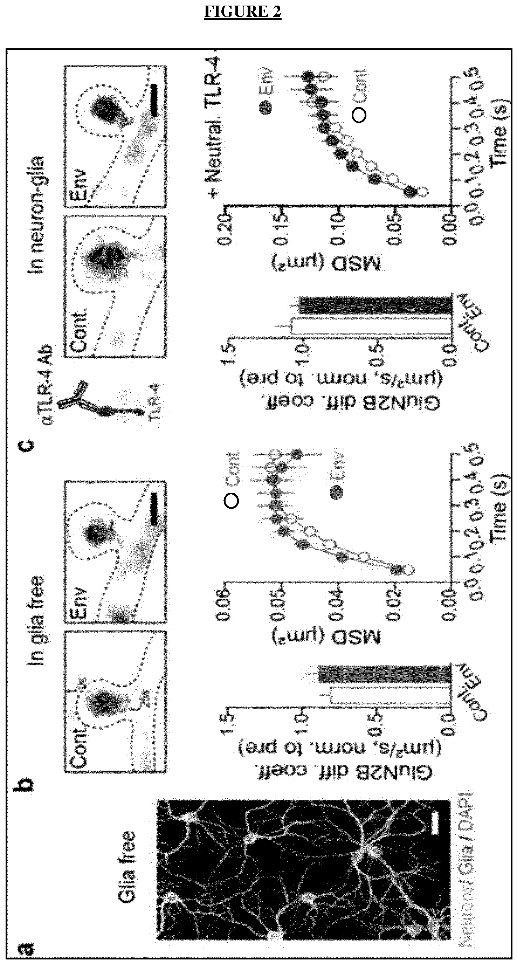

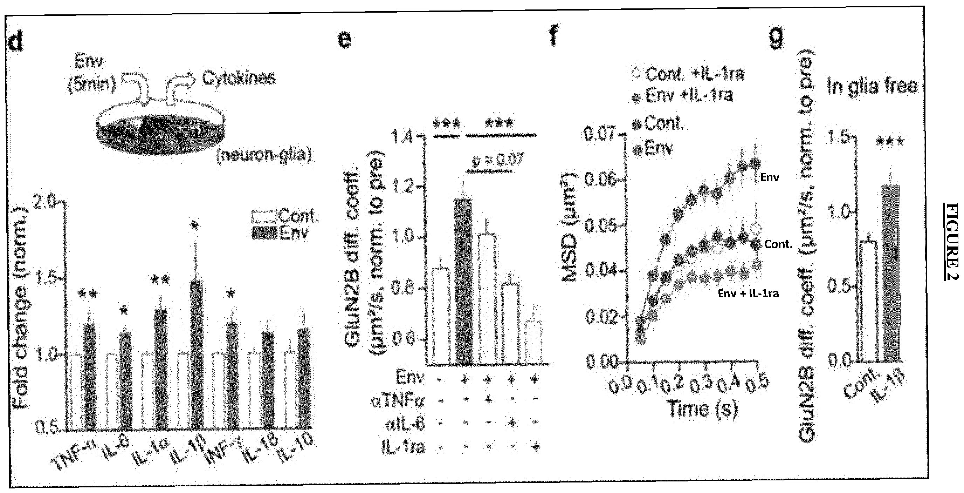

**FIGURE 2****: Glial cells are involved in ENV-induced glutamatergic disturbance.**
   **(a)** Representative image of glia free neuronal cultures (yellow) neurons (MAP-2 positive) and (magenta) glia (GFAP and Ibal positive). Scale bar = 50µm. **(b)** GluN2B synaptic diffusion in glia free cultures after 5min vehicle (Cont., n = 295/7 trajectories/neurons) or ENV (1µg/ml, n = 377/6) exposure. (right) Mean square displacements (MSDs). **(c)** GluN2B synaptic diffusion after Cont. or ENV exposure in the presence of a TLR-4 neutralizing-Ab. Cont. (n = 163/5), ENV (1µg/ml, n = 276/6), (right) MSDs. Scale bars = 1µm. **(d)** Detected cytokine release in culture medium 5min after ENV (10µg/ml) exposure, (n = 5 cultures) *P = 0.037, 0.026, 0.026, **P = 0.009, 0.009, paired Student's t-tests. **(e,f)** GluN2B synaptic diffusion in presence of ENV (1µg/ml) plus αTNF-α, αIL-6 (1µg/ml) blocking Ab's or IL-1receptor antagonist (IL-1ra, 250ng/ml). Cont. (vehicle, n = 579/6), ENV (n = 352/6), ENV + αTNF-α (n = 461/5), ENV + αIL-6 (n = 404/4), ENV + IL-1ra (n = 365/7). ***P < 0,0001 and 0.006, Kruskal-Wallis test followed by Dunn's multiple comparisons. (f) MSDs for data in E including Cont. + IL-1ra (n = 176/7). **(g)** Increased GluN2B surface diffusion after 5min IL-1β exposure in glia free hippocampal cultures. Cont. (n = 423/7), IL-1β (1ng/ml, n = 480/7). ***P < 0,0001, Mann-Whitney test. Data (mean ± SEM) are normalized to pre-exposure for individual neurons.

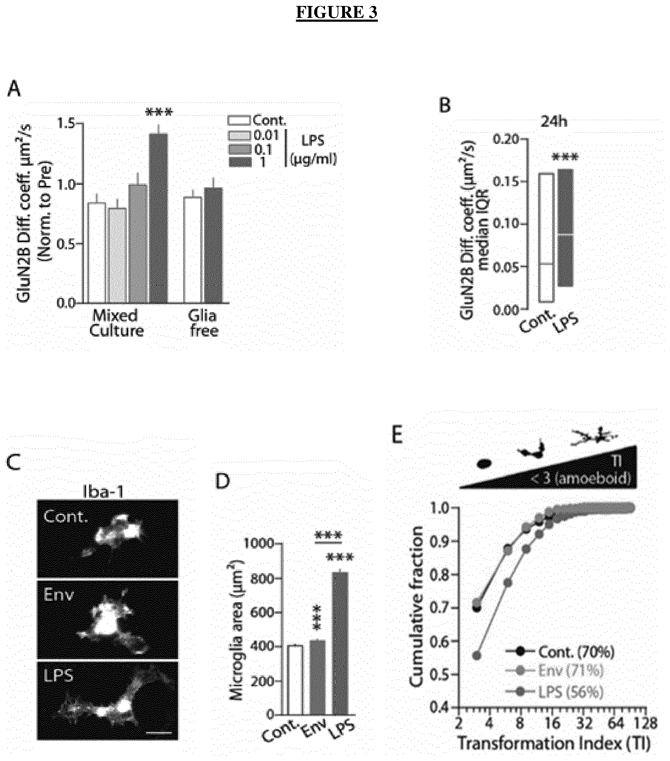

**FIGURE 3****: GluN2B response to LPS stimulation and diverse microglial activation compared to ENV.**
   **(A)** Increase in synaptic GluN2B-NMDAR diffusion coefficient in mixed not glia free cultures 5 min after LPS (1µg/ml) stimulation. Mixed cultures : Saline (Cont.) n = 340/6 (trajectories/neurons), LPS (0.01µg/ml) n = 281/5, LPS (0.1µg/ml) n = 329/5, LPS (1µg/ml) n = 446/6. Glia free cultures: Cont. n = 547/4, LPS (1µg/ml) n = 312/4. Data are normalized to baseline-condition, before addition of Cont. or LPS for each individual neuron ± SEM. ***P = 0,0004, Kruskal-Wallis test followed by Dunn's multiple comparisons. **(B)** GluN2B-NMDAR synaptic diffusion after 24h LPS stimulation. Cont. n = 990/27, ENV n = 606/32. Data represent median ± 25-75% inter-quartile range (IQR), ***P < 0,0001, Mann-Whitney test. **(C)** Representative images of Ibal positive microglia cells 24h after LPS (1µg/ml) or ENV (1µg/ml) exposure. Scale bar = 10µm. **(D)** Quantification of microglial cell areas (µm²) in mixed cultures blank (Cont.) n = 1420 (cells), ENV n = 1279 and LPS n = 1763. ***P < 0,0001 from Kruskal-Wallis test followed by Dunn's multiple comparisons. **(E)** (top) Graphic illustration of microglia morphology in relation to transformation index (TI). (bottom) Cumulative fraction of **TI** in the three conditions with **TI** value < 3 defined as amoeboid cells. Note the change in percentage of amoeboid cells after LPS stimuli (56%) compared to Cont. (70%) and ENV (71%).

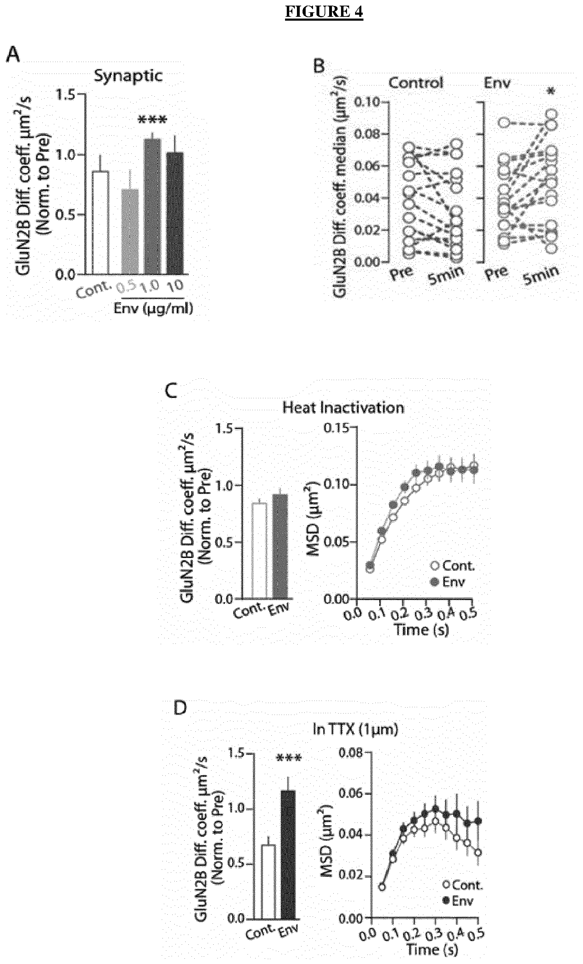

**FIGURE 4****: Synaptic GluN2B response to ENV**
   **(A)** Synaptic GluN2B-NMDAR diffusion coefficient 5min after ENV application at different doses. Data shown are normalized to baseline, before addition of vehicle (Cont.) or envelope-protein (ENV) for each individual neuron, mean ± SEM. Cont. n = 164/19 (trajectories/neurons), ENV 0.5µg/ml, n = 90/7, ENV (1µg/ml), n = 805/13, ENV (10µg/ml), n = 140/7. ****P* <0.0001 from Kruskal-Wallis test followed by Dunn's multiple comparisons.
   **(B)** Median diffusion coefficient for synaptic GluN2B-NMDAR trajectories from individual neurons (circles). Paired data is shown for baseline (pre) and 5min after Vehicle (Cont. n = 16/12 neurons/cultures), or ENV (1µg/ml, n = 17/13) addition. **P* = 0,038, two-tailed paired t-test. **(C)** Heat inactivation abolishes the ENV effect. Heat inactivated vehicle (Cont., n = 563/7) or ENV (n = 443/6). *(right)* Mean square displacements (MSDs) for synaptic data. **(D)** Co-application of tetradotoxin (TTX, 1µM) and ENV do not abolish the ENV effect (Cont., n = 181/3) or ENV (n = 182/4). Data shown are diffusion coefficient normalized to precondition for each individual neuron, mean ± SEM.

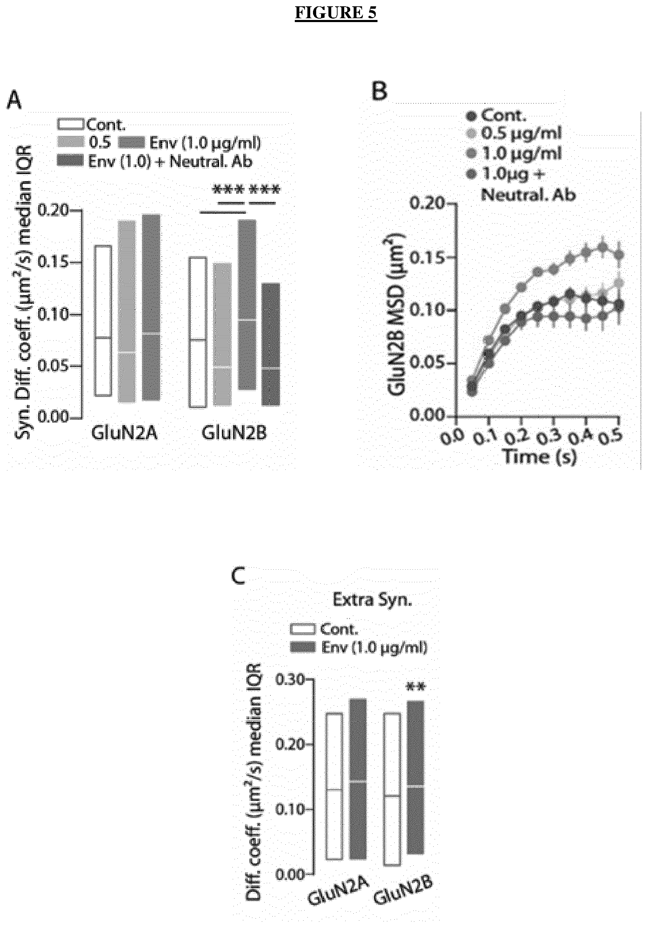

**FIGURE 5****: GluN2B response to prolonged ENV exposure and ENV-specific effects neutralized by anti-HERV-W ENV antibody.**
   **(A)** Specific increase of GluN2B-containing NMDAR-receptors surface mobility in response to 24h ENV exposure. *GluN2A:* Cont. n = 382/15 (trajectories/neurons), ENV(0.5µg/ml) n = 147/6, ENV(1.0µg/ml) n = 277/13. *GluN2B:* Cont. n = 612/22, ENV(0.5µg/ml) n = 485/19, ENV(1.0µg/ml) n = 600/18, anti-HERV-W ENV Ab (N.Ab) n=204/14. ****P* = 0,0004, Kruskal-Wallis test followed by Dunn's multiple comparisons. **(B)** Mean square displacements (MSDs) for data in (A). **(C)** Extrasynaptic surface diffusion of GluN2A- and GluN2B-NMDAR after 24h ENV exposure. *GluN2A:* Cont. n = 2214/28 (trajectories/neuronal fields) ENV n = 1670/31, *GluN2B:* Cont. n = 3882/65, ENV n= 3872/51. **P = 0,0029, Mann-Whitney tests. Values represent synaptic median diffusion coefficient ± 25-75% inter-quartile range (IQR).

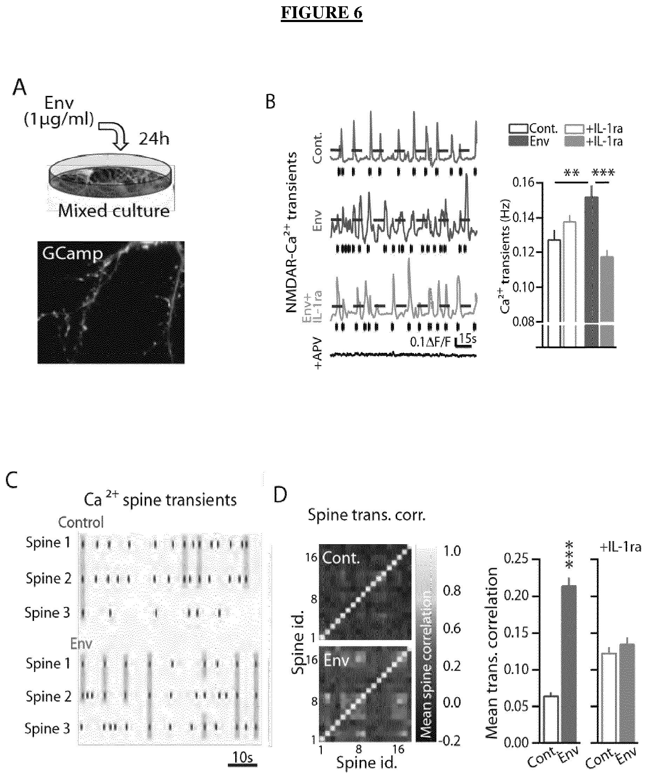

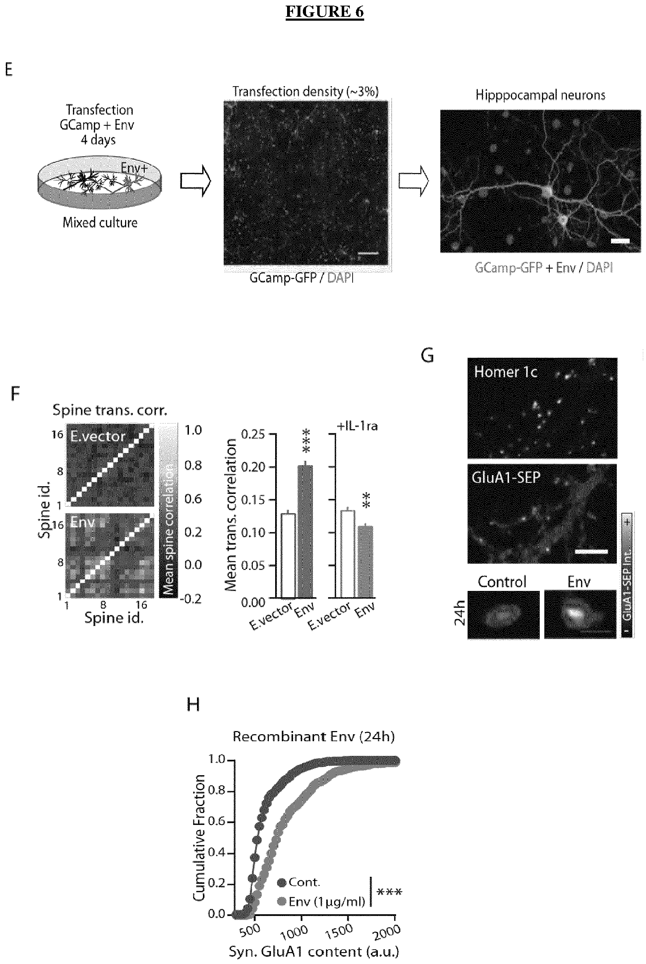

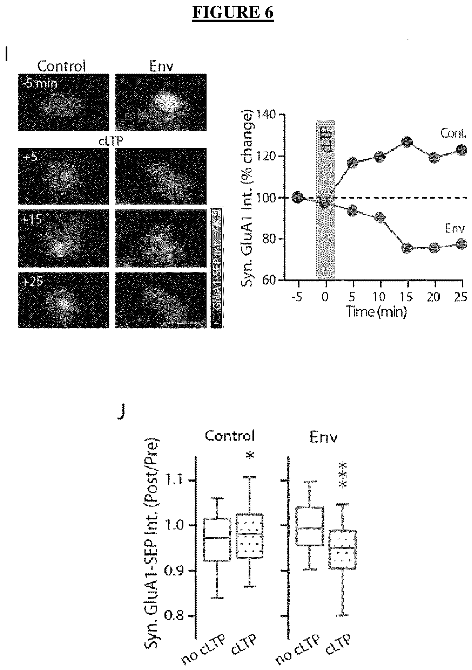

**FIGURE 6****: HERV-W ENV (ENV) induces alterations in glutamatergic network activity and synaptic actions.**
   **(A)** Experimental setup. Scale bar = 10µm. **(B)** An increase in NMDAR-mediated Ca²⁺ transient frequency after long-term (24h) exposure to ENV is modulated by IL-1ra co-exposure. Cont. (n = 103/8 spines/neurons), Cont.+IL-1ra (n = 110/7), ENV (n = 79/10), ENV+IL-1ra (n = 111/7). ***P* = 0.0055 and ****P* < 0.0001, two-way ANOVA followed by Bonferroni's multiple comparisons. **(C)** Ca²⁺ transient detections (dots) and correlated timing (shaded areas) from 3 spines on two example neurons after long-term Cont. and ENV exposure. **(D)** Characteristic correlograms of mean spine transient correlation after 24h Cont. or ENV exposure and *(right)* quantitative graphs. ****P* < 0.0001, Mann-Whitney tests. **(E)** Experimental setup and representative images of co-transfected neurons. Scale bars = 300 and 40µm. **(F)** Correlograms and quantitative graphs from Cont. (empty vector) and ENV (phCMV-MSRV ENV) transfected cells in combination, or not, with IL-1ra. Cont. n = (118/8), Cont.+IL-1ra (n = 151/8), ENV (n = 166/8), ENV+IL-1ra (n = 154/8), ****P* < 0.0001 and ***P* = 0.0052, Mann-Whitney tests. (**G**) Surface GluA1-SEP in synaptic areas (Homer 1c). Scale bars = 5 and 1µm. (***G**(bottom)* and **H**) Synaptic GluA1 fluorescence intensity after prolonged ENV exposure, Cont. (n = 1071/25, spines/neuronal fields) and ENV (n = 728/21), ****P* < 0.001, Kolmogorov-Smirnov test. **(I)** Live time-lapse images at baseline (-5min) and after chemical LTP (cLTP) induction and *(right)* example of synaptic GluA1-SEP fluorescence evolution after cLTP induction, normalized to baseline, for Cont. and ENV. Scale bar = 1µm **(J)** GluA1-SEP intensity ratio between post cLTP and baseline (pre) for individual spines. Box-and-whisker plot (10-90 percentile) with Cont. *no cLTP* (n = 621/13), *cLTP* (n = 902/12), ENV *no cLTP* (n=461/17), *cLTP* (n=472/14). **P* = 0.0233, ****P <* 0.0001, Mann-Whitney tests. Data represent mean ± SEM if no other mentioned.

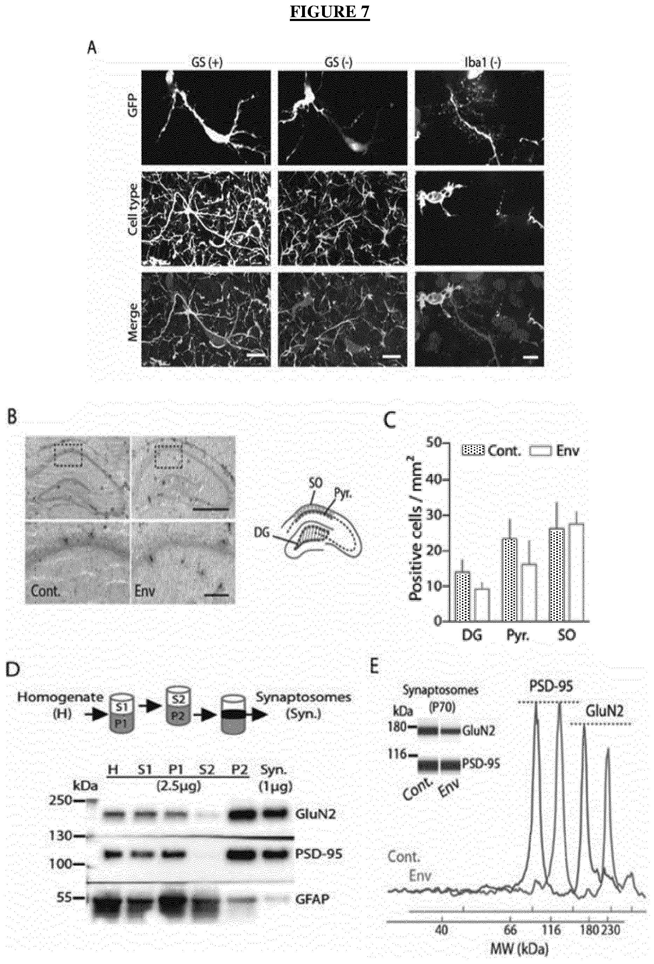

**FIGURE 7****: Expression of MSRV-ENV in electroporated cells, cell viability and GluN2 expression in hippocampal synaptosome fractions.**
   **(A)** Coronal sections of hippocampal CA1 areas from GFP-electroporated animals at P7, co-stained for glutamine synthetase (GS) or ionized calcium-binding adapter molecule 1 (Iba1). Scale bars = 10µm. **(B)** Representative images of apoptotic cell detection (TUNEL) in hippocampal CA1 areas from electroporated animals at P7. Scale bars = 1mm and 200µm. **(C)** Quantification of positive cells / mm² in different regions. Values represent mean ± SEM, n = 3 animals/group. **(D)** Western Blot analysis of subcellular fractionation. NMDA receptors (GluN2A/2B) and the postsynaptic density protein (PSD-95) are enriched in P2- and synaptosome fractions compared to H, whereas depletion of glia (GFAP) is observed. **(E)** Representative chemiluminescent traces from synaptosomes probed on a WESTM-apparatus for GluN2(A+B) and PSD-95.

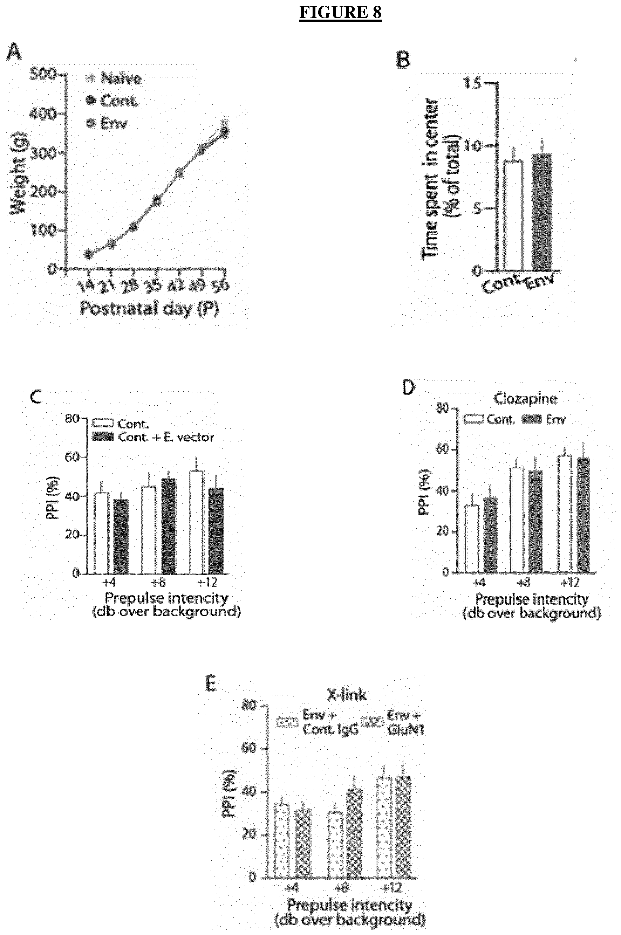

**FIGURE 8****: Effects of DNA insertion on weight development and behavioral performance**
   **(A)** Normal weight gain in electroporated animals compared to naive animals (n = 17-31 animals/group). **(B)** The Env-rats show no alteration in anxiety-like behaviour measured as time spent in centre of the open field (n = 16). **(C)** No influence of DNA load on pre-pulse inhibition (PPI) response (Cont. n = 11, Cont. + Empty vector n = 14). **(D)** Clozapine (12mg/kg) improves the PPI response in Env animals (n = 12). **(E)** PPI response in Control-animals after crosslink protocol with either Cont. IgG or GluN1-Ab (Cont. IgG n = 12, GluN1 n = 11). Values are mean ± SEM.

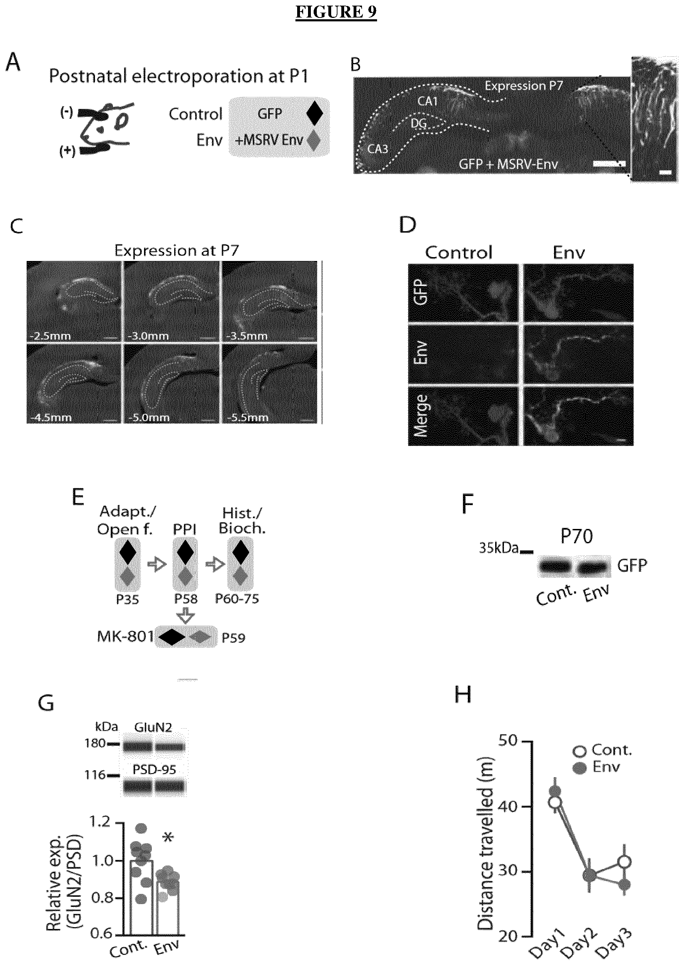

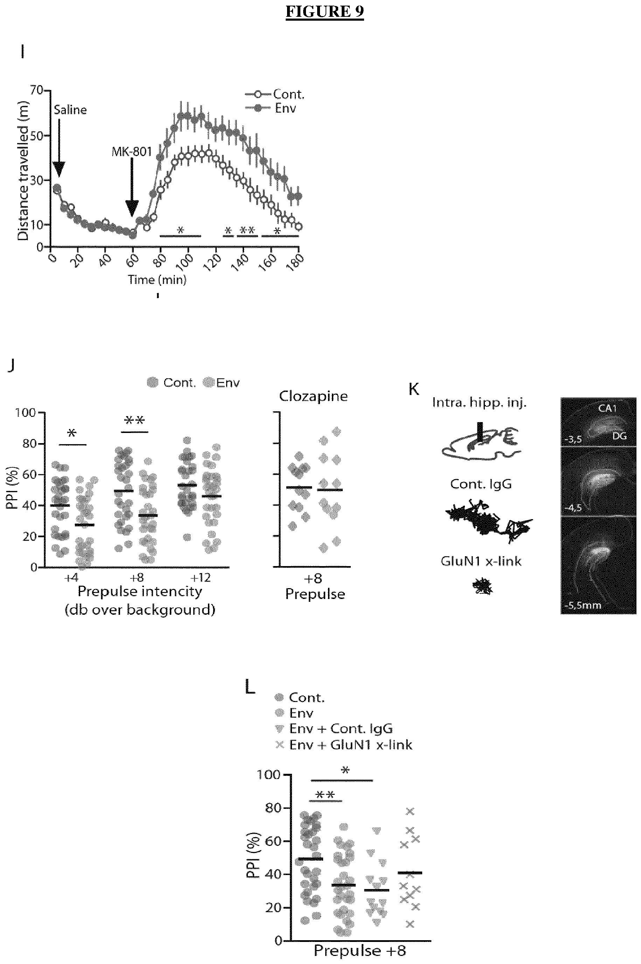

**FIGURE 9****: Selective HERV-W ENV (ENV) insertion in hippocampal cells induces synaptic NMDA alterations and behavior associated to psychosis in rats.**
   (**A**) Schematic representation of electroporation at postnatal day (P)1. (**B** and **C**) Representative images of hippocampal inserted genes distribution. Scale bars = 1mm and inset = 100µm. (**D**) phCMV-MSRV ENV expressing cell from the CA1 region. Scale bar =20µm. (**E**) Behavioral study sequence: Adaptation (Adapt.)/Open field, pre-pulse inhibition (PPI) and NMDAR antagonist (MK-801) challenge. (**F**) Blot of inserted gene expression (GFP) 70 days after electroporation. (**G**) Decrease in GluN2(A+B)/PSD-95 ratio in ENV-hippocampal synaptosomes. **P* = 0.012, Mann Whitney test. Dots are individual animals and bars represent means. (**H**) Basal locomotion and adaptation to context measured as distance traveled (cm) during 20min. Values represent mean ± SEM. (**I**) Increase in MK-801 (0.3mg/kg) induced hyperlocomotion in ENV animals. Values represent mean ± SEM, **P* and ***P* (black lines), RM two-way ANOVA, Fisher's LSD post-hoc analysis, (n = 9-11 rats/group). (**J**) ENV expression impairs PPI startle response. **P* = 0.0180 and ***P* = 0.0019, two-way ANOVA, followed by Bonferroni's multiple comparisons test. *(right)* Clozapine (12mg/kg) recovers the disrupted PPI response. (**K**) Experimental design, *(right)* intra-hippocampal injection site. (**L**) PPI performance in ENV animals after GluN1 x-linking. **P* = 0.0195, ***P* = 0.0093, one-way ANOVA corrected for unweighted means, followed by Tukey's multiple comparison test. Dots are individual animals and lines represent means.

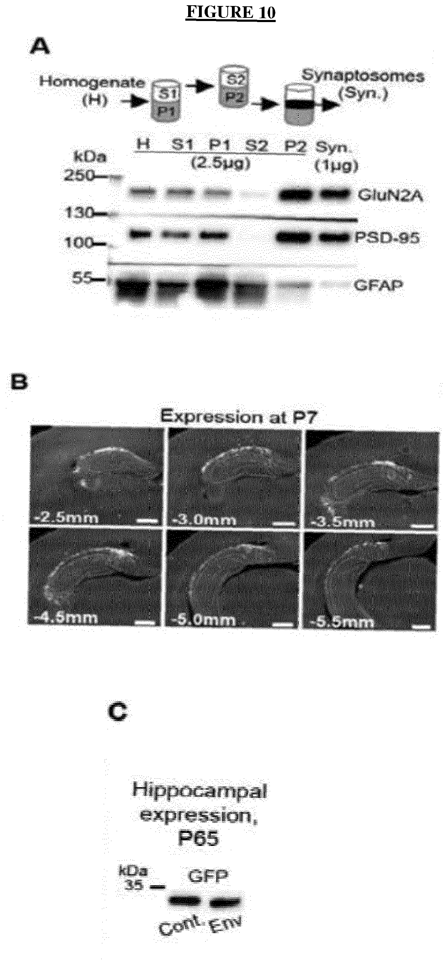

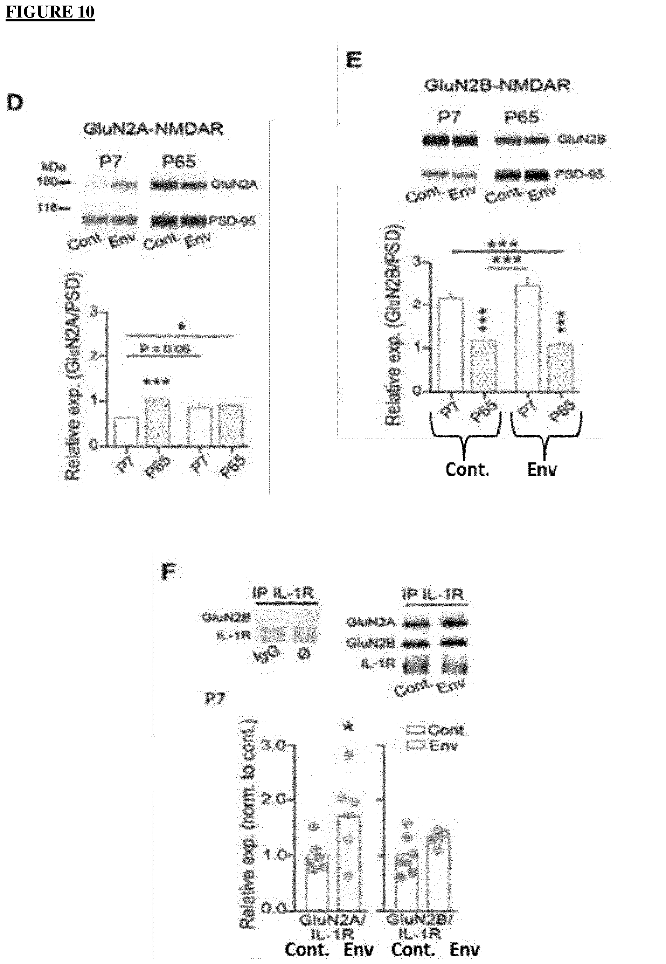

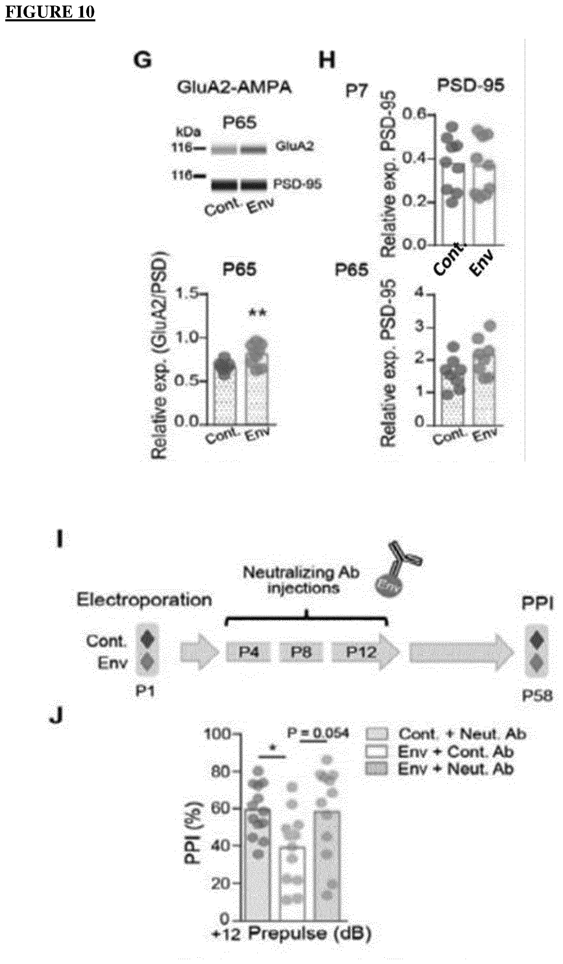

**FIGURE 10****: Neonatal ENV expression tunes glutamatergic synapse maturation and is necessary for psychotic-like behavior, which can be reversed by in vivo injections of HERV-W ENV neutralizing antibody.**
   (A) Subcellular fractionation of hippocampal tissue. Note the enrichment of NMDA receptors (represented by GluN2A) and post-synaptic density proteins (PSD-95) in P2- and synaptosome (synaptic proteins enriched) fractions compared to initial homogenate and, the depletion of glia (GFAP) in the same. **(B)** Representative images of inserted genes distribution at P7. Scale bars = 1mm. **(C)** Hippocampal inserted gene expression at ~P65. **(D)** ENV exposure tends to influence GluN2A/PSD-95 subunit stabilization in the synapse (P7, Cont. vs ENV, P = 0.06) which then remains constant. Interaction: F(1,29) = 8.69, two-way ANOVA, Bonferroni's multiple comparisons test, *P = 0.021, ***P = 0.0004. **(E)** GluN2B/PSD- 95 expression is unaffected by ENV at both ages. Interaction: F(1,29) = 2.46, P = 0.13, two-way ANOVA, Bonferroni's multiple comparisons test, ***P < 0.0001, (n = 8-9 animals/group). Bars are mean ± SEM. **(F)** At P7 ENV-animals exhibit increased GluN2A-IL-1R interactions. Immunoprecipitation (IP) of IL-1R, *P = 0.0462, Student's t-test (n = 5-7 animals/group). **(G)** GluA2/PSD-95 expression is increased in adult animals, **P = 0.0098 Students t-test (n = 6-9 animals/group). **(H)** PSD-95 expression is similar comparing Cont. and ENV from both P7 and P65 rats. **(I)** Early postnatal treatment protocol, (i.p. injections) at P4, 8 and 12 with an ENV neutralizing-Ab (30mg/kg). **(J)** Treatment improves ENV-animals PPI responses, *P = 0.037, one-way ANOVA, Tukey's multiple comparison test. Dots are individual animals and bars represent means.

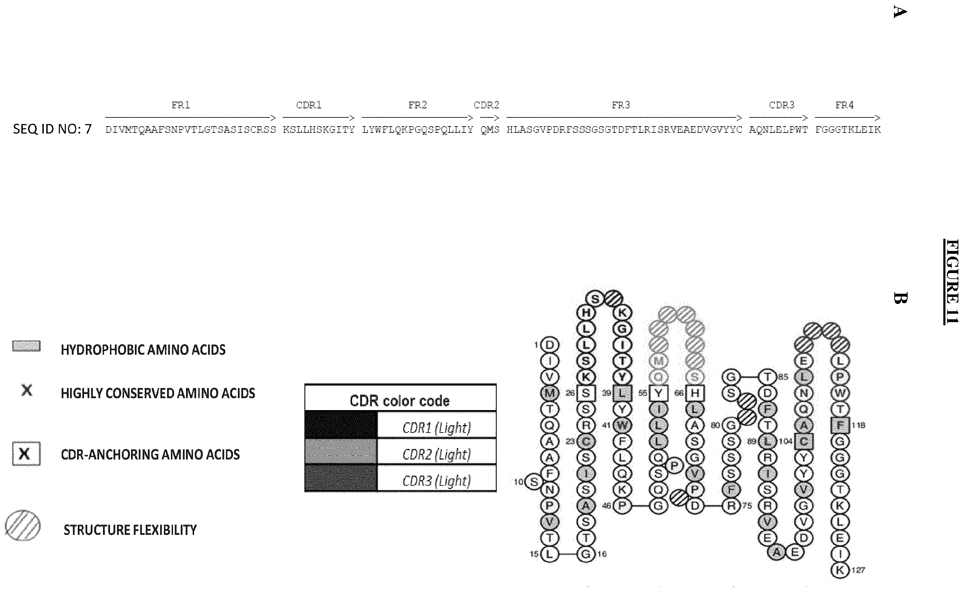

**FIGURE 11****: Functional protein structure with CDR sequences of ENVW2.3 antibody-VL region**
   **(A)** The linear peptide sequence is represented with indication of CDR sequences (CDR1, CDR2 and CDR3). **(B)** "Pearl Necklace representation" according to Pommié C et al., 2004. Journal of Molecular Recognition, 17, 17-32; IMGT (The international ImMunoGeneTics information system for immunoglobulins or antibodies, T cell receptors, MH, immunoglobulin superfamily IgSF and MhSF).

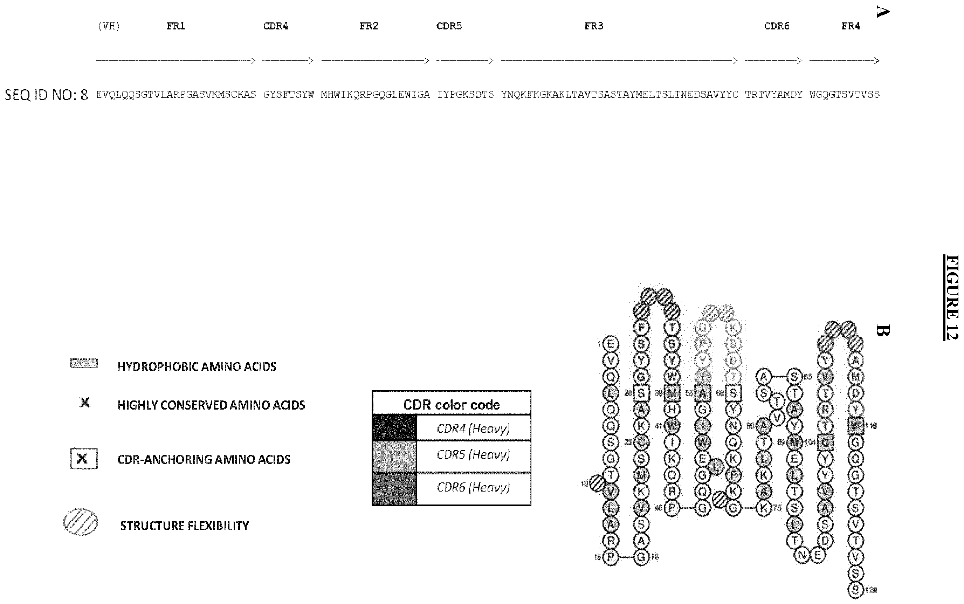

**FIGURE 12****: Functional protein structure with CDR sequences of ENVW2.3 antibody - VH region**
   **(A)** The linear peptide sequence is represented with indication of CDR sequences (CDR4, CDR5 and CDR6). **(B)** "Pearl Necklace representation" according to Pommié C et al. 2004. Journal of Molecular Recognition, 17, 17-32; IMGT (The international ImMunoGeneTics information system for immunoglobulins or antibodies, T cell receptors, MH, immunoglobulin superfamily IgSF and MhSF).

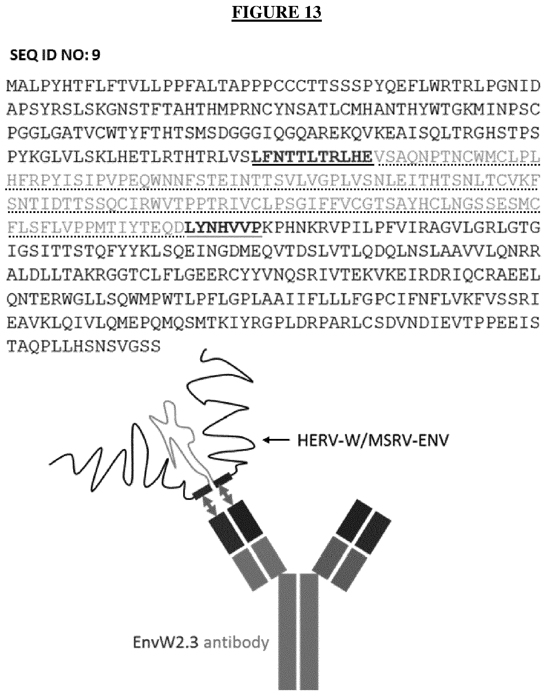

**FIGURE 13****: HERV-W/MSRV-ENV amino acid sequence with ENVW2.3 fixation site underlined with a black solid line (site 1) and with a grey solid line (site 2).**
   Tertiary conformation of MSRV-ENV protein induces the sequences underlined with a black solid line and with a grey solid line (site 1 and site 2) to be butted against one another. This generates the conformational epitope recognized by ENVW2.3 antibody. The sequence underlined with a dashed line corresponds to amino acids contained in-between the site1 and site 2 forming the ENVW2.3 epitope but is not recognized by the antibody.

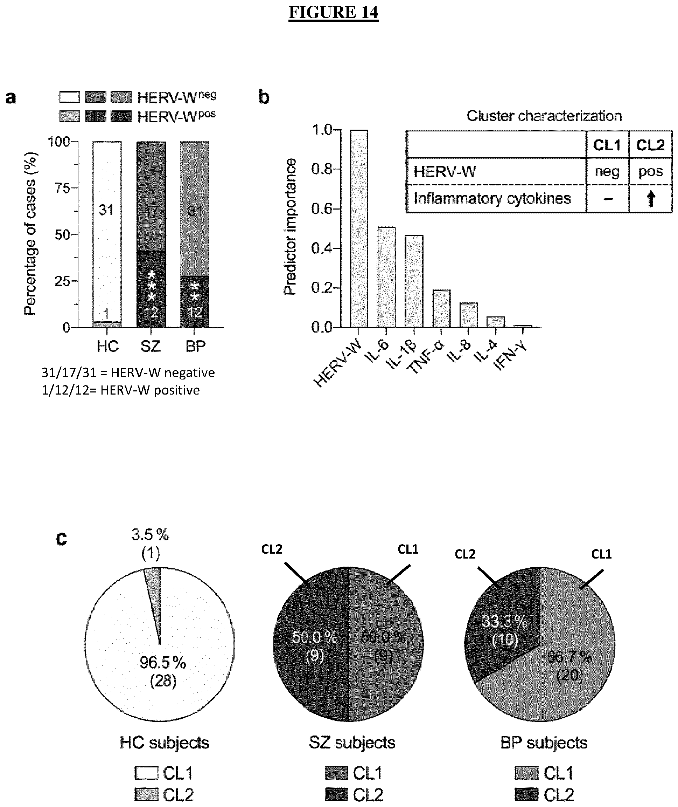

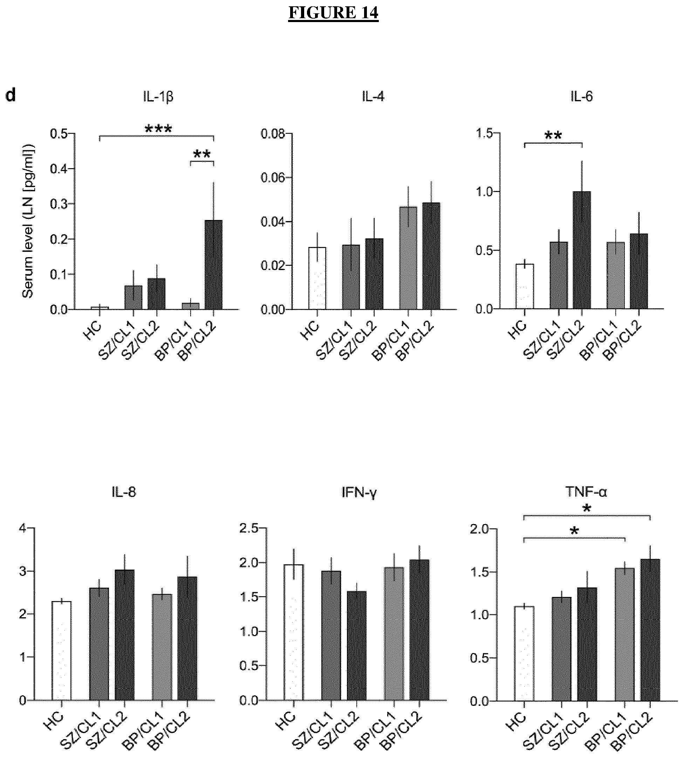

**FIGURE 14****: HERV-W ENV expression and inflammatory cytokines in serum define subgroups in schizophrenia and bipolar disorder.**
   **(a)** Percentage of healthy controls (HC), cases with schizophrenia (SZ) and cases with bipolar disorder (BP) who are positive (HERV-Wpos) or negative (HERV-Wneg) for HERV-W ENV antigenemia. Embedded numbers represent the number of subjects. **p < 0.01 and ***p < 0.001, reflecting the significant difference between HC and SZ or BP cases, based on Chisquare tests. **(b)** Predictor importance for cluster stratification and summary of cluster characteristics as revealed by two-step cluster analysis. Cluster analysis revealed two main clusters (CL1 and CL2), which were separated based on HERV-W positivity and serum cytokines. CL2 subjects are HERV-Wpos and show increased levels of serum cytokines, whereas CL1 subjects are HERV-Wneg with no overt changes in serum cytokines. **(c)** Distribution (percentage, %) of HC, SZ and BP subjects across CL1 and CL2. The numbers in brackets represent the number of subjects in each cluster. **(d)** Serum levels of interleukin (IL)-1β, IL-4, IL-6, IL-8, interferon (IFN)-y and tumor necrosis factor (TNF)-α in HC subjects, subgroups of SZ cases (SZ/CL1 and SZ/CL2) and subgroups of BP cases (BP/CL1 and BP/CL2). *p < 0.01, **p < 0.01 and ***p < 0.001, based on Tukey's post-hoc tests for multiple comparisons following one-way ANOVA. The bars represent means +/- S.E.M.

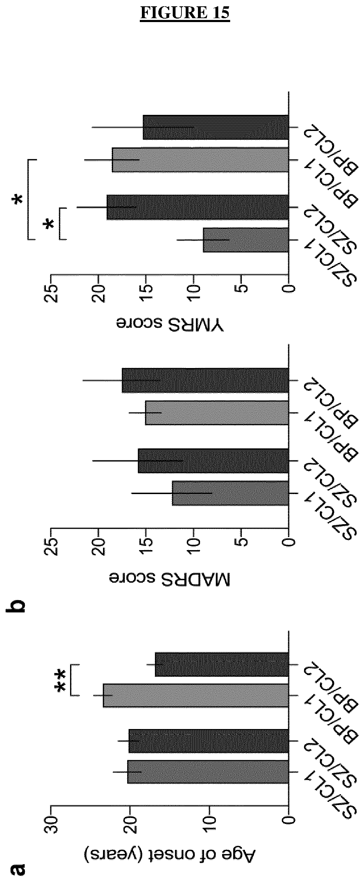

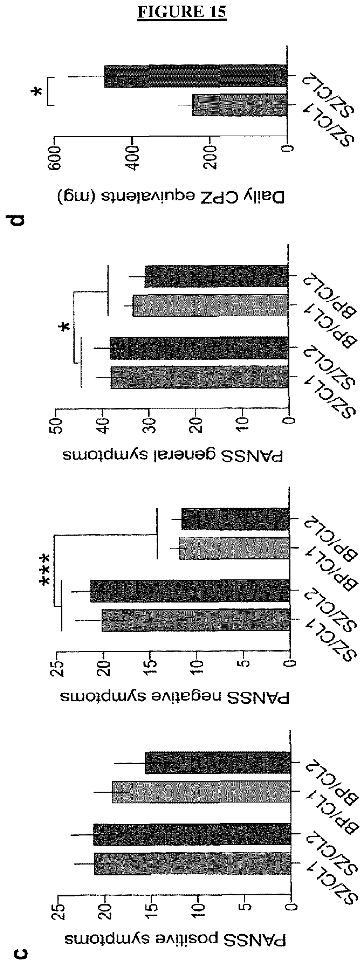

**FIGURE 15****: Distinct subgroups of schizophrenia and bipolar disorder cases show differing clinical characteristics.**
   Cluster analysis revealed two main clusters (CL1 and CL2), which were separated based on HERV-W positivity and serum cytokines (see Figure 14). CL2 subjects are HERV-Wpos and show increased levels of serum cytokines, whereas CL1 subjects are HERV-Wneg with no overt changes in serum cytokines (see Figure 14). (a) Age of disease onset (years) in the subgroups of SZ cases (SZ/CL1 and SZ/CL2) and subgroups of BP cases (BP/CL1 and BP/CL2). **p < 0.01, based on Tukey's post-hoc tests for multiple comparisons following two-way ANOVA. **(b)** Montgomery-Asberg Depression Rating Scale [MADRS] scores and Young Mania Rating Scale [YMRS] scores in the subgroups of SZ cases (SZ/CL1 and SZ/CL2) and subgroups of BP cases (BP/CL1 and BP/CL2). *p < 0.05, based on Tukey's post-hoc tests for multiple comparisons following two-way ANOVA. **(c)** Positive and Negative Syndrome Scale [PANSS] scores (positive, negative and general symptoms) scores in the subgroups of SZ cases (SZ/CL1 and SZ/CL2) and subgroups of BP cases (BP/CL1 and BP/CL2). *p < 0.05 and ***p < 0.001, reflecting the significant difference between SZ and BP subjects regardless of subgroup classification, based on two-way ANOVA. **(d)** Daily chlorpromazine [CPZ] equivalents in subgroups of SZ cases (SZ/CL1 and SZ/CL2). *p < 0.05, based on tow-tailed t-test. All values are means +/- S.E.M.

### Materials and Methods for examples 1 to 3

### Study Design.

We designed this study to determine whether HERV-W derived envelope-protein (ENV) could play a role in the known glutamatergic, namely NMDAR, disturbance of developmental neuropsychiatric disorders. We first showed that the ionotropic function of the receptor (NMDAR antagonists are related to psychotic like effects) was intact after ENV exposure and that instead, the surface diffusion of the NMDAR-GluN2B subtype was sensible to ENV. The recombinant ENV buffer was used as control and in addition, heat inactivation and neutralization with an specific ENV-antibody proved involvement of HERV-W ENV At the commence of single particle tracking experiments the sample size was on the one hand, determined by referring to previous studies from the laboratory and on the other hand, calculated (Power & Sample Size Calculator, Statistical Solutions, LLC) with a power factor of 0.6-0.8 and α of 0.5 which in our condition was translated to 4-13 cells per condition depending upon the SD of the sample. For all type of experiments a minimum of 3 independent cultures were used per condition. Cultures were chosen at random and experimental conditions were alternated throughout the live imaging experiments, the experimenter was blind to the condition during removal of unspecific trajectories while all other analyses were done on a fully automated basis. Immunocytochemistry showed that the expression of the important synaptic GluN2A/2B NMDAR-subunit balance was affected. All analysis involving immunohistochemistry/immunocytochemistry were performed blindly. We controlled that no toxicity was induced by ENV in our neuronal networks by adding Propidium Iodide and visualizing healthy cell bodies post ENV exposure. We next measured the global NMDAR-mediated activity, as well as on NMDAR-dependent long-term synaptic potentiation (LTP) and observed an increase in calcium frequency and ablation of cLTP induction in the ENV treated cultures. In the chemical LTP experiments a variation above 10 % of GluA1SEP intensity in single synapses during baseline were considered as unstable and hence this spine was excluded, for calcium imaging, spines with more than 2 calcium burst after D-APV application were considered as non NMDAR and excluded. We then transfected neuronal cultures with a plasmid containing the HERV-W ENV and observed similar results. An plasmid with an empty vector was used as control. We then asked if specific ENV expression could affect animal's behavior. For this, we expressed the ENV in the hippocampus of rat pups from postnatal day 0 and studied its impact on a series of behavioral studies. We controlled for cellular toxicity by evaluating TUNEL stained cells and weight gain of the animals and, the presence and extent of ENV expression was confirmed by immunohistochemistry and western blot analysis. Expression of GFP and additionally an empty vector was used in control animals. For behavioral studies the predetermined minimum sample size of 12 animals was based on previous studies. In our hands, a sample size of 13-18 animals in PPI were used, which corresponded to a power factor of 0.6-0.75 and α of 0.5 and in the x-link and MK-801 experiments a sample size of 11 animals corresponded to a power factor of 0.55 and <0.8 respectively. Rat pups from the same litter were assigned to different experimental groups in a randomized manner and groups were pseudo-randomized on each behavioral testing day. As predetermined, potentiation of PPI in response to prepulses and in the MK-801 challenge test, stereotypic behavior were used as exclusion criteria's. During animal studies the experimenter was not blind to the animal's condition since all analysis were done on a fully automated basis at the end of the experiment, after collecting the complete data set. As we observed deficits in several behavioral tests we then investigated the animal's hippocampal NMDAR expression in order to relate our in vitro data to a possible underlying cause for these observations. Protein expression studies were conducted with 9 animals per condition and replicated 2-3 times. By specifically neutralizing the ENV during development we observed a clear improvement of the behavioral response. The data showed that HERV-W ENV expression in the hippocampus induce behavioral deficits and modulate the synaptic NMDAR maturation during development.
Expression of the ENV in serum from 7 patients with neuropsychiatric disorders was detected by ELISA. Serum samples from 6 individuals with no known brain disease were used as controls and sample sizes were based on the availability of samples. In a pilot experiment, we stimulated neuronal networks transfected with plasmids containing the main neurotransmitter receptors (AMPA, NMDA, Dopamine and GABA) with our serum samples and observed that ENV containing serum specifically induced NMDAR alterations.

### ELISA multiplex

Cytokine levels were examined using a Milliplex Map Kit (RECYTMAG-65K, Millipore). Culture medium, from 3 different cultures, collected 5 min after vehicle (Control) or ENV (10µg/ml) application were processed according to the manufacturer's recommendations and mean fluorescent intensities were obtained using a Luminex xPONENT software on a Bioplex™ MAGPIX reader (BioRad, Hercules, USA). Data was normalized to control within each experiment.

### Envelope Protein.

Recombinant ENV (full-length MSRV envelope protein of 548aa; ENV pV14; GenBank accession no. AF331500) was produced by PX'Therapeutics (Grenoble, France) according to quality control specifications of (GeNeuro Geneva, Switzerland). Endotoxin removal was done by polishing batches through Mustang Q Acrodisc followed by filtration on 0.22µm filter Stericup (Merck, Darmstadt, Germany). Endotoxin levels for ENV batches used were between 13.6-92.3 EU/ml as measured by the limulus amebocyte lysate test. Influence of endotoxin on our results was excluded by observations after heat inactivation, 100°C for 30 min.

### Receptor surface diffusion experiments.

Neurons were transfected with the postsynaptic marker Homer 1c-DsRed at 7 div. High resolution single molecular tracking of NMDAR's were achieved after 10 min incubation at 37°C with antibodies against extracellular epitopes of either the GluN2A or the GluN2B subunits (Alomone Labs, Jerusalem, Israel, Table S3) at 11-14 div. After 10 min incubation with Quantum dots (QDs) 655 (Invitrogen, Thermo Fisher Scientific, Massachusetts, USA) in medium with 1% BSA (Sigma-Aldrich, Missouri, USA), selected regions of interest (ROI)s (with Homer-1c expressing neurons) were imaged for 500 consecutive frames with an acquisition time of 50 ms on a Nikon eclipse Ti epifluorescent microscope using an EMCCD camera (Evolve, Photometric, Tuscon, USA). Acquisition was made with MetaMorph software (v.7.7.11.0, Molecular Devices, Sunnyvale, USA). The instantaneous diffusion coefficient (D) was calculated for each trajectory from linear fits of the first four points of the mean square displacement (MSD) versus time function using MSD(t) = < r2 > (t) = 4Dt. To determine the distribution of single QD complexes, frame stacks were obtained and after binarisation of the synaptic signal the complexes were automatically located into synaptic (Homer-1c positive area including surrounding 2 pixels) and extrasynaptic compartments. The percentages of synaptic locations per stack in relation to the total amount were calculated and the two highest deltas (pre-post) were excluded as outliers from each group. Data were projected on a single background image, providing high-resolution distribution of receptor/QD complexes and their trajectories. All single particle analysis was completed using the Palmtracer v1.0 plugin in MetaMorph software (Molecular Devices). The effect of ENV (GeNeuro) or LPS (Sigma-Aldrich) compared to respective vehicle (Controls) on receptor surface diffusion were addressed using 2 approaches: i) after 5 min bath application in the imaging chamber following an initial baseline acquisition or, ii) 24h after protein application to the culture medium in the dish. Recombinant IL-1β (R&D Systems, Minnesota, USA) were only evaluated after 5 min application. TLR-4 involvement was studied by blocking the receptor through pre-incubation for 30 min with an antiTLR-4 neutralizing Ab (20µg/ml, Affymetrix, Wien, Austria) at 37°C before onset of QDexperiment. The specificity of the TLR-4 neutralizing antibody was confirmed by principal TLR4 staining on Iba-1 positive microglia cells (Fig. S2A) and decreased staining after shTLR-4 transfection (plasmid kindly provided by Dr. Küry, Fig. S2B). To confirm the specificity of our ENV results on the one hand, heat inactivation of the protein was performed (see above) and on the other, pre-incubation of the recombinant ENV protein with ENV-neutralizing antibody, GN_ENV_01/03 (Geneuro), in normal horse serum at a molecular weight ratio (1:2) was performed in glass tubes for 45 min at R.T. before application. For isolation of a postsynaptic response tetradotoxin (1µM, TTX, Tocris, Bristol, UK) was applied into the chamber before recordings and for cytokine blocking experiments, IL-1ra (250ng/ml, R&D Systems) or, the neutralizing antibodies IL-6 and TNF-α (1µg/ml, R&D Systems) was applied into the chamber before recording sessions. The involvement of Src-family kinases was evaluated after 15 min incubation with 1 µM PP2 (Calbiochem, Merck).

### Cell cultures and transfection

Mixed cultures of hippocampal neurons and glia cells were prepared from E18 Sprague-Dawley rats. In brief, cells were plated at a density of 300-350x100 cells per dish on poly-lysine coated coverslips and were maintained in Gibco neurobasal medium (Thermo Fisher Scientific, Massachusetts, USA) containing 3% horse serum for approximately 4 days in vitro (div) at which the medium were changed to a serum-free neurobasal medium. Banker type "glia free" hippocampal cultures were prepared in two steps. Briefly, first glia feeder cultures were prepared in poly-lysine coated dishes from hippocampus then, after two weeks, hippocampal neurons (from the same type of preparation as for the glia cells) were cultured on poly-lysine coated coverslips which were suspended above the glia layer. Cells were kept at 37°C in 5% CO2 for 22 div at maximum. Human embryonic kidney cells (HEK) 293 were plated on glass coverslips in Dulbecco's modified Eagle's medium (Thermo Fisher Scientific) with 10% fetal calf serum and used one day later.

Cells were transfected using either Effectene (Qiagen, Hilden, Germany) according to the manufacturer's recommendations or by phosphate calcium transfection. The plasmid encoding HERV-W ENV protein consisted in the reference MSRV-env gene inserted into a phCMV vector allowing expression in transfected human cells: phCMV-MSRV env from GeNeuro, Switzerland. The inserted env synthetic nucleic acid sequence is encoding HERV-W envelope protein as described in databases (Genbank Ref. AF 331500).

### HERV-W ENV Protein

Recombinant ENV (full-length MSRV envelope protein of 548 aa; ENV pV14; GenBank accession no. AF331500) was produced by PX'Therapeutics (Grenoble, France) according to quality control specifications of GeNeuro (Geneva, Switzerland). Endotoxin removal was done by polishing batches through Mustang Q Acrodisc followed by filtration on 0.22µm filter Stericup (Merck, Darmstadt, Germany). Endotoxin levels for ENV batches used were between 13.6-92.3EU/ml as measured by the limulus amebocyte lysate test. Influence of endotoxin in our results was excluded by observations after heat inactivation, 100°C for 30 min as previously described (18).

### Immunocytochemistry

Before human serum incubation, neurons (10 div) were transfected with either the α-amino-3-hydroxy-5-methyl-4-isoxazolepropionic acid receptor (AMPA)-A1 containing; the N-Methyl-D-Aspartate receptors (NMDA)-N1 containing; the gamma-Aminobutyric acid (GABA)-γ2 containing subunits fused to the Super Ecliptic pHluorin (SEP) or the dopamine (D)-1 containing subunit fused to Cyan Fluorescent Protein (CFP). Serum samples were slowly thawed and following incubation, with ENV-positive or ENV-negative serum samples at 20% for 15 min at 37°C, live immunostaining of surface receptors were conducted with an anti-GFP antibody for 10 min at 4°C (heat inactivation of complement factors in serum samples was considered but not applied due to the known heat sensitivity of the ENV-protein). Live staining was followed by 15 min fixation in 4% paraformaldehyde (PFA). In general, fixation was followed by quenching in 50mM NH4Cl, blocking and incubation with secondary Ab's coupled to Alexa fluorophores for 1h in 1% bovine serum albumin (BSA) (Sigma-Aldrich, Missouri, USA) at room temperature (R.T.). For staining of fixed cells and tissues incubation overnight with primary antibodies at 4°C was done after an initial block and permeabilization step in 1% BSA (Sigma-Aldrich) and 0.1% Triton X-100 (Sigma-Aldrich) for 30 min. Cells were mounted in Mowiol (Calbiochem (Merck)) or Vectashield® + DAPI (Vector Laboratories, Burlingame, CA). Surface or intracellular staining of ENV in HEK-cells transfected with cytosolic enhanced green fluorescent protein (EGFP) and MSRV-ENV was obtained after 48h with an anti-ENV antibody ((GN-mAb 01; GeNeuro Switzerland),). For the microglia activation study, cells were fixed and stained for ionized calcium-binding adapter molecule 1 (Iba1) 5min after LPS (serotype 026:B6, Sigma-Aldrich) (1µg/ml), ENV (PX'Therapeutics) (1µg/ml) or Vehicle (Control) application. Images were either collected on a video confocal spinning-disk system (Leica DMI6000B, Wetzlar, Germany) with a CoolSNAP HQ2 camera (Photometries, Tucson, USA) or, on a Nanozoomer (Hamamatsu, Japan). Cluster area was obtained using a fixed threshold approach based on integrated fluorescence levels in ImageJ. Microglia perimeter (µm) and area (µm2) was obtained from all microglia present on entire coverslips and a transformation index (TI) was calculated (perimeter of cell) 2/4pi (cell area) based on previous publications (27).

### Calcium Imaging.

Neurons transfected with GCaMP3 or GCaMP6 at 10 div were transferred into Tyrode's solution containing (in mM): 110 NaCl, 5 KCl, 25 HEPES, 15 D-glucose, 2 CaCl2 and 2 MgCl2. For isolation of NMDAR dependent transients the neurons were then moved to Mg²⁺ free Tyrode's with 5µM Nifedipine (Tocris) and 5µM Bicuculline (Tocris) 15min before imaging. Time-lapse images were acquired with MetaMorph software (Molecular Devices) at 20Hz on a Nikon eclipse Ti epifluorescent microscope with an EMCCD camera (Evolve, Photometric). To asses immediate effects tree time lapse movies (3000 frames) were successively recorded: Pre (baseline), Post (5 min after bath application of PBS (Blank), Vehicle (Control) or ENV and, APV (5 min after 50µM D-(-)-2-Amino-5-phosphonopentanoic acid (APV, Tocris) bath application). For prolonged effects two time lapse movies were recorded. The first, 24h after Vehicle (Control), ENV (1µg/ml), Vehicle + IL-ra (250ng/ml) or ENV + IL-ra addition or, 4 days after transfection with the ENV-gene or an empty-vector, and the second, 5 min after APV bath application. Time-lapse movies were concatenated and realigned in ImageJ (NIH) with the PoorMan3DReg plugin (Michael Liebling). Fluorescence from calcium transients vs. time was measured within individual ROIs (spines) manually defined by the experimenter (ImageJ, NIH). All pixels within each ROI were averaged to give a single time course associated to the ROI. Mean normalized fluorescence (ΔF/F) was calculated by subtracting each value with the mean of the previous 5s values lower than P50 (µ) and dividing the result by µ. Positive calcium transients were identified following a two-step procedure: initially, ΔF/F traces were smoothened by convoluting the raw signal with a 10s squared kernel. Using custom-written Matlab routines, true positive NMDA transients (with minimum 1 sec between transients) were defined on an automated basis where the threshold was set at 5*SD of APV average trace. Pairwise cross-correlation of transients between spines on the same neuron was computed using a time window of 0.5 seconds. We corrected the correlation values by subtracting the mean correlation obtained by shuffling the inter-transient time for individual spines (repeated 100 times).

### Chemically induced potentiation (cLTP).

Live hippocampal neurons, co-transfected at 10 div with GluA1-SEP and Homer 1c-DsRed, were incubated (12 div) overnight with ENV (1µl/ml) or nothing (Control) at 37°C. Chemically induced long-term potentiation (cLTP) was provoked by bath co-application of 200 µM Glycine (Tocris) and 5 µM Picrotoxin (Tocris) for 4 min as previously described (24). cLTP was always applied after a period (2 x 5 min) of baseline acquisition and the medium was carefully replaced by fresh equilibrated and heated medium after induction. GluA1-SEP fluorescence signal was then recorded every 5 min during the following 30 min. Synapses were outlined using the synaptic Homer 1c signal and GluA1-SEP intensity (ImageJ, NIH) was followed over time within these synaptic areas and then exclusively normalized to baseline if synapses showed a stable baseline (variation below 10% between - 10 and -5 min). All images were collected on a video confocal spinning-disk system (Leica, DMI6000B, Wetzlar, Germany) with a CoolSNAP HQ2 camera (Photometrics, Tucson, USA).

### Animals.

Pregnant rats (Sprague Dawley) were purchased from Janvier (France) and on P 0-1 male pups from the same litter were assigned to different groups in a randomized manner: Control, Control+ or ENV. Rats were kept at constant ambient temperature (21±1°C) with ad libitum access to food and water. Every effort was made to minimize the number of animals used and their suffering. Animal procedures were conducted in accordance with the European Community guidelines (Directive 2010/63/EU) regulating animal research, and were approved by the local Bordeaux Ethics Committee (APAFIS#3420-2015112610591204). Postnatal electroporation was done in newborn pups between PO-1, as previously described. Briefly, pups were anesthetized by hypothermia and injected with deoxyribose nucleic acid (DNA) constructs coding for cytosolic EGFP to identify transfected cells (Control-group) in combination with an empty vector (Control+ -group) or, with phCMV-MSRV ENV (clone pV14, AF 331500) (ENV-group). Approximately 2 µg of DNA in 8 µl of PBS and 0.1 µl of Fast Green were injected into lateral ventricles, immediately followed by electroporation with five electrical pulses (150 V, 50-ms duration, 1-s interval between pulses) delivered by a pulse generator (BTX, Harvard apparatus, ECM830, San Diego, CA). Pups were reanimated on a thermal blanket at 37°C and quickly returned to their mother.

### Behavioral responses.

Experiments were conducted during the light cycle (14.00-20.00) by the same experimenter as the one who handled the animals during their lifespan. Their weights were monitored every week and at P 21 the pups were weaned and housed 2-5 rats from the same litter and with the same treatment/cage. Five different study-groups were used: i) the animals in the first group, Control (n = 13), ENV (n = 13), were subjected to a series of behavioral tests in the following order: (1) at P35-37, locomotor activity and anxiety, (2) habituation/adaptation to the open field and (3) at P56-58 the prepulse inhibition test (PPI) and 2 days after (4) social recognition and interaction. ii) The animals in the second study-group, Control (n = 30), ENV (n = 30), was either exposed to Clozapine/Vehicle treatment before PPI or/and to a MK-801 challenge on the day after PPI. iii) The third study group, Control (n = 11), Control+ (n = 14), was tested in the PPI and, iv) the fourth study-group, ENV (n = 22), was subjected to a crosslink protocol (see below for stereotaxic injection) on the day before PPI. v) The animals in the last group Control (n = 13), ENV (n = 24), were treated with the ENV neutralizing-Ab during an early postnatal period. All animals were naive to the apparatuses when first presented with the tests and acclimatized to the room for at least 1h before onset. Groups were pseudo-randomized on each behavioral testing day. During the open field and PPI tests the experimenter was not blind to the animal's condition although behavioral data collection was done using a fully computer controlled setup and in an unbiased way. In contrast, the investigator was blinded to the treatment during scoring analysis in the social recognition and interaction test. See figure 4C for experimental layout.
***Open field.*** Locomotor activity was measured in an open field arena (54cm long x 54cm wide x 40cm high) with light settings at approximately 5 lux. Novelty-induced locomotion was assessed by video tracking the rat which was allowed to freely explore the empty arena during 2x10 min on day 1 out of 3 consecutive test days. From the recordings on day 1, anxiety was evaluated as the time spent within a center zone comprising 50% of the arena during the first 10 min. Then, habituation/adaptation to context was assessed as a decrease of locomotor activity on day 2-3. For MK-801 studies, Control (n = 13) and ENV (n = 12) rats were given an intraperitoneal (i.p.) injection with saline and left to explore the arena during one hour. Then, a MK-801 (0.5mg/kg, Tocris) injection was administered (i.p.) and animals were monitored for two additional hours. One animal was excluded due to stereotypic behavior after injection. Total distance travelled was extracted with the IDtracker software (43) and analyzed with automatized custom-written MATLAB routines.

### PrePulse Inhibition (PPI).

PPI was performed using a Panlab startle chamber (Harvard, San Diego Instruments). Each PPI session lasted for approximately 31 min and began with a 5 min acclimatization period with a constant background noise. The session consisted of 8 different trial types: a no pulse, a startle pulse (120 decibel (dB) at 8kHz, 40ms) that was or, was not preceded by tree prepulses at +4, +8, and +12 dB above a 74 dB background noise (20ms, interval 100ms) and, the prepulses alone. Each session started with 10 startle pulses (ITIs 70sec) followed by a counterbalanced pseudorandom order of the 8 trials x 6 and ended by a final block of 10 startle pulses. Baseline data from different groups were initially pooled seeing that the effect of Envgene insertion on the overall PPI measured at the three prepulses was similar: 1) two-way ANOVA, group factor; F(1,72) = 11.23, **P = 0.0013, n = 13 and, 2) two-way ANOVA, group factor; F(1,102) = 9.597, **P = 0.0025, n = 18. Potentiation in response to the prepulses was observed in both animal groups and these animals were excluded from the final data set. Prepulse inhibition is expressed as % PPI and was calculated by (100*((S-PP)/S), where S = average response on startle-only trials and PP = average response on prepulse + startle trials. We confirmed that the DNA load was trivial to the behavioral outcome (study-group iii) by comparing GFP animals with GFP + empty vector electroporated animals (Fig. S6C). In study-group ii) Clozapine (Abcam, Cambridge, UK) (12 mg/kg) was dissolved in 0.1 N HCl and buffered with NaOH to pH 5.6 and a single (i.p.) injection (1 ml/kg, clozapine or vehicle) was administered to Control (n = 12) and ENV (n = 12) animals 20 min before PPI evaluation (Fig. S6D). Vehicle treatment did not affect PPI responses (data not shown). Stereotaxic injections were conducted in study-group iv) one day before the PPI test. Briefly, rats (P55-57) from the ENV-group were anesthetized with isoflurane and placed in a stereotaxic frame. 0.8mg (2µl) of polyclonal goat anti-rabbit IgG (Novex, Thermo Fisher) (ENV + Cont.IgG) (n = 13) or anti-rabbit GluN1 (Alomone Labs) (ENV + GluN1 crosslink) (n = 11), diluted in 0.1% Tryptan Blue (Sigma-Aldrich) (1:5), were stereotaxically injected into each hippocampus (coordinates relative to bregma, AP: -4.5 mm, ML: ±3.2 mm, DV: -2.0 mm). Based on the cross-study coherence, the increased response to increased prepulse in all groups and strong response at prepulse +8 dB, PPI data obtained from this prepulse is preferentially shown. Complete data set can be observed in Fig. S6E. Experiments from study group's ii-iv were executed in parallel. Early postnatal treatment was conducted by a single i.p. injection of the ENV-neutralizing Ab (GN_mAb_ENV_03, 30mg/kg, GeNeuro) at PN4, 8 and 12, animals were then left in their home cages until the day of PPI test at ~P58.

### Social recognition and interaction.

This protocol was adjusted from (43). The day before testing, 3 rats (1 study rat (Control or ENV), and 2 target rats (naive)) with similar weight (± 5%) from different litters were assigned to be tested together. Target rats were then habituated to the experimental cage (a housing cage) in the experimental room during 1h. At the day of experiment, the study rat was habituated to the experimental cage for 1h. Thereafter, without any training the test started and the first target rat was placed into the same experimental cage (3x1 min, ITI 3 min) and social recognition was recorded. Subsequently during a last interaction session the first target rat was removed and the second target rat was placed into the experimental cage together with the study rat and social interaction was recorder for 10 min. In the last session, control study rats showed increased interaction during the first minute meaning that the rats were once again interested in the unfamiliar rat (second target rat). The full last session (10 min) was then scored in order to measure social interaction. Study and target rats were not used in this paradigm more than one time. Videos were scored in a blinded fashion for the time the study rat actively engaged in social interacting behaviors (sniffing, grooming, close following, and crawling over/under). No aggressive behaviors were noted during the sessions.

### Tissue Preparation

Brains were removed between 2-14 days after PPI and either the whole brain; was rapidly frozen in isopentane (Sigma-Aldrich) placed in liquid nitrogen or, transferred to ice cold artificial cerebral fluid for dissection of the hippocampal areas and then frozen in liquid nitrogen. The frozen hippocampal tissue was later processed for Western blot as described below. For immunohistochemistry, 20 µm thick coronal tissue sections were cut on a microtome-cryostat, thaw-mounted onto superfrost ultra plus (Thermo Scientific) slides, and stored at -20°C until further processing. Animals at the age P7, Control (n=5) ENV (n=5) and, P59-70 Control (n=5) ENV. (n=5) were anesthetized with pentobarbital (50 mg/kg) and transcardially perfused with 4% paraformaldehyde in 0.1M phosphate buffer. Brains were removed and postfixed overnight at 4°C and 50-µm-thick slices were prepared with a VT1200S Leica vibratome. Slices were washed three times with PBS and left in 0.03% acid-PBS at 4°C for later use.

### Immunohistochemistry/TUNEL Staining

Perfused tissue were blocked and permeabilized in BSA (Sigma-Aldrich) and 0.1% Triton X-100 (Sigma-Aldrich) for 2h at R.T. After rinsing samples were incubated with primary Ab's in 2% BSA (Sigma-Aldrich) and 0.2% Triton X-100 (Sigma-Aldrich) overnight at 4°C. Secondary Ab's coupled to Alexa fluorophores were incubated for 2h at R.T. in the same solution as the primary Ab's. Sections were mounted using Mowiol (Calbiochem) or Vectashield® + DAPI (Vector Laboratories, Burlingame, CA). All images were collected on a video confocal spinning-disk system (Leica DMI6000B, 63X) with a CoolSNAP HQ2 camera (Photometrics) or on a Nanozoomer (Hamamatsu).

DNA fragmentation was histologically examined using the in situ Apoptosis Detection System Fluorescein (TUNEL, Promega, Madison, WI). Frozen tissue sections from P7 electroporated animals were stained according to the manufacturer's recommendations and mounted with Mowiol (Calbiochem).

### Western blot analyses

Dissected hippocampus from control and ENV electroporated rats (~P70) were processed by subcellular fractionation (Fig. 6D) to solely collect synapses (synaptosomes), synaptic plasma membranes and synaptic vesicles. Protease and phosphates inhibitors (ThermoFisher Scientific) were added to the isotonic sucrose for homogenization and fractionation. The protein concentration of each sample was determined with Pierce BCA Protein Assay Kit (ThermoFisher Scientific) and synaptosomes from 9 animals per condition were examined on two separate experiments. For GFP detection whole hippocampus homogenates were used. We used a WESprotein simple apparatus (Protein simple, bio-techne, San Jose, USA) and WES-total protein pack, plus WES- standard pack (12-230 kDa) including anti rabbit secondary antibody, antibody diluent, molecular weight ladder, streptavidin-HRP, dithiothreitol (DTT), fluorescent master mix, luminol-S, peroxide, sample buffer and wash buffer plus for GluN2B detection in relation to PSD-95. This kit also provides capillary cartridge and pre-filled microplates. 0.1µg of each sample was loaded and specifics of primary antibodies, rabbit anti-GluN2B (Agrobio) and anti-PSD-95 (Cell Signaling, Danvers, USA). Only values within the range of two standard deviations were included for further analysis. GFP was detected by conventional western blotting. Briefly, 20µg of total protein was loaded in each lane separated with 4-20% SDS-polyacrylamide gel electrophoresis and transferred to a nitrocellulose membrane (Bio-Rad, Hercules, USA). The membrane was blocked in 5% non-fat Milk TBS/0.1% Tween 20 (TBST) at room temperature for 1 h. Primary antibody mouse anti-GFP (Roche, Basel, Switzerland) were diluted in 0.5 % Milk in TBST for protein immunoblot analysis and incubated O.N. at 4°C under agitation. Incubation with HRP-conjugated anti-mouse IgG (Jackson Immunoresearch Laboratories, West Grove, USA) was performed for 2 h at room temperature. Specific protein bands were revealed with Clarity™ Western ECL Substrate (Bio-Rad) and the membranes were scanned using Versadoc (Bio-Rad).

### Preparation of figures and analysis.

Figures were assembled in ImageJ (NIH) and Adobe Photoshop (Adobe Systems, San Jose, CA), only contrast and brightness were adjusted to optimize the image quality. All statistical analysis was performed in GraphPad Prism 6 (GraphPad Software, San Diego, USA). In brief, statistical analysis using alpha = 0.5 were conducted. For behavioral studies a predetermined sample size was based on the literature. Here, a sample size of 13-18 animals were used in the PPI, which correspond to a power factor of 0.6-0.75 and α of 0.5 and, in the cross-link and MK-801 experiments a sample size of 11 animals correspond to a power factor of 0.55 and <0.8 respectively. Parametric statistical tests were applied when data passed the D'Agostino & Pearson omnibus normality test hence, two-tailed Student's t-test with or without Welch's correction, one- or two-tailed paired Student's t-test or two-way ANOVA followed by Bonferroni's multiple comparisons test were conducted. Comparisons between groups with data from non-gaussian distributions were performed using nonparametric test: either the two-tailed unpaired Mann-Whitney test or, for multiple groups the Kruskal-Wallis test followed by Dunn's multiple comparison test. For direct comparison of distributions, Kolmogorov-Smirnov test was used. For behavioral testing, the PPI results were analyzed using the factors group and prepulse by two-way ANOVA followed by Bonferroni's multiple comparisons test for group and prepulse intensity. A one-way ANOVA analysis, followed by Tukey's multiple comparisons test for treatment was used in the crosslink and Neutralizing Ab protocols. RM two-way ANOVA considering the factors treatment and time followed by Fisher's LSD post-hoc analysis were used for analysis of the MK-801 response and a two-way ANOVA for TUNEL data with factors treatment and area. Significance levels were defined as *P < 0.05, **P < 0.01, ***P< 0.001.

### EXAMPLE 1: Effects of sera from psychotic patients on neuronal neurotransmitter receptors.

In a first experiment, we collected sera from patients suffering from psychotic disorders (schizophrenia and bipolar disorder) or from control healthy subjects in order to test their impact on the content of key neurotransmitter receptors in hippocampal neuronal networks containing neurons, astrocytes and microglia (Fig. 1A-C). Irrespective of the glutamatergic AMPA (GluAl subunit) and NMDA (GluN1 subunit), dopaminergic D1, and inhibitory GABA_{A} (gamma2 subunit) receptors, sera from psychotic patients specifically decreased NMDA receptor (NMDAR) clusters (Fig. 1D). Of note, these patients also displayed a high level of the pro-inflammatory cytokine, interleukin-lbeta (IL-1β), when compared to control subjects (Fig. 1A and B). Because we used human sera from psychotic patients, this NMDAR alteration with the specific characteristics evidenced from *in vitro* and *in vivo* studies, as described in the following examples, is consistent with the presence of HERV-W ENV in the tested sera (antigenemia). As also described in the following examples, it is also consistent with the relevance of HERV-W ENV pathogenicity that involves IL-1β and innate immune properties of microglia for the specific dysregulation of neuronal synaptic NMDAR function. Most importantly, it is also consistent with the discovery of a novel nosocomial entity represented by a sub-group of patients gathering cases with diagnosed schizophrenia or bipolar disorder and elevated pro-inflammatory cytokines detection in serum, including IL-1β. Patients included in this novel biologically-defined nosological entity had been diagnosed either with schizophrenia or with bipolar disorder.

### EXAMPLE 2: HERV-W ENV triggers specific cytokine release from microglia with synergistic neuropathogenic effects.

Surprisingly, when glia-free neuronal cultures (without astrocyte or microglia) were exposed to recombinant ENV, GluN2B-NMDAR synaptic dynamics remained stable (Fig. 2A and B). In the presence of microglia in the neuronal cultures, the pro-inflammatory bacterial lipopolysaccharide molecule, LPS, was capable to rapidly disperse synaptic GluN2B-NMDAR (Fig. 3A and B). However, microglial cells activated either by LPS or ENV displayed different phenotypes (Fig. 3C-E), evidencing that these two proteins activate different signaling pathways despite their common affinity for TLR4, which results in effects of different natures and mechanisms on the neuronal cells. In search of the glial mediator that was released by glial cells triggered by ENV, we measured *in vitro* a high release of pro-inflammatory cytokines, especially IL-1β, in the medium of cultures reproducing hippocampal networks (Fig. 2 D). To test whether these cytokines, especially IL-1β, played a direct role in the synaptic NMDAR destabilization by ENV, hippocampal networks were exposed to ENV together with various cytokine blockers. The IL-1ra (250ng/ml), a natural IL-1 receptor (IL-1R) antagonist, reversed the ENV-induced GluN2B-NMDAR synaptic dispersal. Blocking IL-6 (1µg/ml) or TNF-α (1µg/ml) only partly reduced the effect and did not yield a full blockade (Fig. 2E). Furthermore, IL-1β alone was sufficient to increase GluN2B-NMDAR trafficking in glia-free neuronal cultures (Fig. 2G). Thus, these data provide the first evidence that HERV-W ENV is able to rapidly disperse synaptic NMDAR through a glial- and cytokine -dependent process. This is now shown to represent a novel mechanism by which HERV-W ENV can alter neuronal functions by affecting neuroreceptor distribution in synapses via interleukin-1, possibly synergizing with IL-6 and/or TNF-α, but targeting a specific subclass of neuroreceptor (GluN2B-NMDAR). Moreover, despite its possible induction by multiple proinflammatory agonists such as LPS, the observed effects of ENV-induced cytokines from microglial cells also revealed an ENV-specific effect on the GluN2B-NMDAR trafficking in and around neuronal synapses.

The functional alteration of neuronal functions by HERV-W ENV therefore reveals to be highly specific, at both levels of glutamatatergic neuroreceptor subclass and of microglia-mediated inflammatory cytokine production. Thus, pro-inflammatory cytokines and the most relevant one, IL-1β, involved in this specific neuronal dysfunction, are not induced by environmental factors such as infectious agents but by the HERV-W ENV protein itself when expressed and/or released in a neuron-glia tissue environment. Since no such specific effect of IL-1β, alone or in association with IL-6 and/or TNF-α, has ever been described nor could be expected, it becomes obvious that the contextual induction and synergistic effects of HERV-W ENV are key to this ENV-specific pathogenic mechanism.

This also unravels the existence of a specific biological continuum, based on these ENV-glia/cytokine-mediated neuronal dysfunctions, in psychiatric diseases classified in different diagnostic categories until now, such as Schizophrenia and Bipolar Disorder.

Present nosological entities only rely upon psychological evaluations with dedicated tests and scales, as well as on the clinical, mostly behavioral, symptomatology.

The present elucidation of this HERV-W ENV-induced neuronal dysfunction is therefore providing a causal, mechanistic and biological definition that underlies symptoms and disease evolution across different diagnostic categories associated with HERV-W ENV and, IL-1β and/or 11-6 and/or TNF-α, cytokine-driven synergistic effects on brain cells.

A new biologically-defined nosological entity can therefore comprise sub-groups of patients with Schizophrenia, with Bipolar Disorder or with other present psychiatric diagnoses, when resulting from the presently discovered synergistic effect between HERV-W ENV and such cytokines. Depending on upstream, probably environmental and temporal factors (*11*), this HERV-W expression may be activated in various brain regions, in which neuronal synapses with GluN2B-NMDAR are involved in various behavioral, cognitive and other nervous system functions. This also implies neurodevelopmental impairment when HERV-W ENV and cytokine-driven synergistic effects occur during embryogenesis and early life development of brain areas and functions. This unexpectedly unravels a previously unknown but common pathogenic process between different nosocomial entities of present psychiatric diagnoses.
One major aspect of HERV-W ENV-induced pro-inflammatory cytokines is the presence of Il-1 and/or of related pro-inflammatory cytokines, IL-6 and/or TNF-alpha, as observed to be co-expressed in the presently described experiments, but also detectable in body fluids of these patients (cf. Example 5).

### EXAMPLE 3: Specific effect of HERV-W ENV on the NMDAR can be inhibited by monoclonal antibody in vitro and in vivo.

Most psychomimetic drugs (e.g. PCP) are NMDAR antagonists. Despite its biochemical nature, i.e. a protein macromolecule that cannot have the molecular interactions of the small chemical compounds used to interfere with receptors of neuronal neurotransmitters, HERV-W ENV blocked NMDAR-mediated synaptic transmission in hippocampal networks.

An effect of HERV-W ENV protein on neuronal receptors was never described nor could be expected, since only known to interact with TLR4 (30) receptor and V-beta chains of T-cell receptor (*31*), which are not present nor expressed in neuronal cells. Moreover, these results also provided the evidence that HERV-W ENV did not interfere with neuronal receptors through presently known mechanisms since neither NMDAR-mediated calcium transients, nor NMDAR-mediated evoked EPSCs, were altered by various concentration of HERV-W ENV (Fig. 1E-G), indicating that it does not act as a NMDAR antagonist like known psychomimetics. Thus, we discovered that HERV-W ENV specifically altered the NMDAR trafficking in synapses, which could explain the observed effects with both patients' sera and recombinant or tissue-expressed HERV-W ENV protein. To further explore a possible explanation of such non-conventional effects, the two main NMDAR subtypes in the forebrain, i.e. the GluN2A- and GluN2B-containing NMDARs, were tracked in real time at the surface of living neurons using single nanoparticle (Quantum Dot) imaging (Fig. 1H) (32). HERV-W ENV exposure rapidly increased GluN2B-, but not GluN2A-NMDAR, membrane dynamics and mean square displacement (MSD) in the postsynaptic compartment (Homer 1c-positive area; Fig. 1I-J, and Fig. 4). Hence, the presence of HERV-W ENV protein in the neuron culture medium, induces the delocalization of the NMDAR, in particular of the GluN2B-containing NMDA receptor, from the synapses to the postsynaptic compartment (i.e., in neuronal membrane regions out of the synapses) leading to a surprising dispersal of the receptor away from synapses with an original pattern, since also different from that induced by another TLR4-binding molecule, i.e. LPS.

A specific antibody directed against HERV-W ENV was found to inhibit this pathogenic effect (Fig. 1K), which revealed a specific effect targeting the pathogenic potential of this protein on such neuronal receptors. In parallel, heat-inactivated HERV-W ENV (Fig. 4C) showed no significant effect versus control, thereby confirming its pathogenic properties under a native protein conformation. Because this HERV-W ENV-induced effect was still observed in neuronal networks deprived of action potentials by tetradotoxin (TTX) (Fig. 4D), the presently discovered effects collectively demonstrate that HERV-ENV laterally disperses synaptic NMDAR in a subunit-specific and neuronal activity-independent manner, which is not part of the present knowledge on neuropathogenic molecules that interfere with receptors of neurotransmitters (33-35).

To test whether TLR4 activation could unexpectedly trigger this effect, a well-known TLR4 agonist, lipopolysaccharide (LPS), was applied onto hippocampal networks. Consistently, LPS was capable to rapidly disperse synaptic GluN2B-NMDAR (Fig. 3A and B). But LPS, which always had effects similar to that of HERV-W ENV through TLR4 activation (*30*, *36*, 37), here revealed to display a different phenotype than the one induced by HERV-W ENV (Fig. 3C-E), suggesting that these two proteins do not share the same signaling pathways when considering this original effect on neurotransmitter receptors of neurons. The HERV-W ENV protein induces the delocalization of the GluN2B-containing NMDA receptor, from the synapses to the postsynaptic compartment (i.e., in neuronal membrane regions out of the synapses) leading to a surprising dispersal of the receptor away from synapses with an original pattern differing from that induced by LPS.

As schizophrenic patients may be chronically exposed to HERV-W ENV (11), we investigated how long-term exposure to ENV alters hippocampal network properties and animal behavior. After 24h of ENV exposure, synaptic GluN2B-NMDAR were still laterally dispersed, an effect that was fully blocked by a selected anti-ENV antibody (Fig. 5A-C). Functionally, this led to a sustained increase in the frequency of NMDAR-mediated Ca²⁺ transients in dendritic spines (Fig. 6A and B), which became more correlated with each other over time (Fig. 6C-D). These network activity changes were modulated by the blockade of IL-1β (Fig. 6B and D), consistent with previously described observations of ENV-mediated IL-1βinduction in microglia.

Because a bulk exposure of HERV-W ENV may not reflect the bioavailability of the protein in the patients' brains, HERV-W ENV was genetically-expressed for several days in only 3% of hippocampal cells (Fig. 6E). Remarkably, this was sufficient to mimic the overall dispersal of synaptic GluN2B-NMDAR and network effects (Fig. 6F). Finally, these alterations in GluN2B-NMDAR-mediated synaptic and network activities would predict long-term changes in the basal and plastic range of glutamatergic synapses (38, 39). Consistently, the synaptic content of GluA1-AMPA receptor (AMPAR) was strongly increased in neurons exposed to HERV-W ENV (Fig. 6G and 6H). In addition, glutamatergic synapses were unable to undergo chemically-induced long-term potentiation (cLTP). Following the chemical challenge, the AMPAR synaptic content actually decreased, as if synapses underwent de-potentiation (Fig. 6I and 6J). Hence, prolonged HERV-W ENV exposure on hippocampal cells has profound consequences on the network activity and NMDAR-dependent adaptations of glutamatergic synapses. Such major and consistent effects have never been described with HERV-W ENV protein and were not expected to target specific receptors of neurotransmitters at such a specific and significant level, under such peculiar mechanisms.

In psychotic diseases, alterations of the NMDAR signaling and synaptic plasticity have been associated with behavioral deficits in cognitive and sensorimotor gating (i.e. prepulse inhibition, PPI) tests (*40*), which are hallmarks of psychosis models in rodents. Following postnatal electroporation, HERV-ENV electroporated cells were observed in hippocampal astrocyte/radial glial (GS+) or non-glial (GS-) cells (Fig. 7A). Cell survival and body growth were undistinguishable between control (GFP only) and ENV rats (Fig. 7B and 8A). In addition, control and HERV-W ENV+ rats exhibited similar basal locomotion, habituation and context recognition, without behavioral signs of anxiety (Fig. 9H). In adult rats (postnatal day 70), electroporation-induced proteins were still detectable (Fig. 9F) and HERV-W ENV expression decreased the GluN2/PSD-95 ratio in hippocampal synapses (Fig. 9G). Strikingly, ENV-expressing rats displayed an exaggerated hyperlocomotion following a single injection of the psychomimetic drug, MK-801 (NMDAR channel blocker) (Fig. 9I) and their performance in the PPI test was impaired when compared to the one of control littermates (Fig. 9J). Together, these behavioral outcomes unveil that HERV-W ENV expression in the hippocampus is sufficient to trigger behavioral deficits associated to psychosis. In support, the antipsychotic drug, clozapine, prevented the PPI test alterations in HERV-W ENV-expressing rats (Fig. 9J, 8C). Finally, as HERV-W ENV induced NMDAR specific dysfunction through an original mechanism of lateral dispersal, we artificially stabilized surface NMDAR in the hippocampus of HERV-W ENV-expressing rats prior testing their PPI performance using a GluN1 cross-link protocol (Fig. 9K) (*32*, *39*, *41*). Consistently, such a stabilization of membrane NMDAR diminished the HERV-W ENV-induced impairment in the PPI test (Fig. 9L).

Altogether, this study demonstrates that the HERV-W ENV, detected in a large fraction of psychotic patients *(12, 42)*, is capable of generating a dysfunction in the NMDAR organization and long-term plasticity within glutamatergic synapses. This dispersal of the synaptic receptor NMDAR away from the synapses produces behavioral deficits associated to psychosis. This makes HERV-W ENV a therapeutic target of interest for new therapeutic strategies, with novel mechanism of action, in psychotic patients.

It also demonstrates that an anti-ENV antibody could specifically and efficiently reverse and/or prevent these pathogenic effects at the level of a unique neuronal receptor of neurotransmitters, GluN2B when impacted through its biodistribution over neuronal synapses by HERV-W ENV. The use of an anti-ENV antibody induces in neurons, the relocation of the NMDAR, in particular of the GluN2B-containing NMDA receptor, into the synapses. This therapeutic effect of anti-ENV antibody translates, *in vivo,* in the reversal or prevention of psychosis-related behavioral abnormalities.

This interplay between HERV-W ENV and glutamate synapse thus adds a novel and unexpected layer in our understanding of how endogenous retroviruses could play an instrumental role in psychiatric diseases. Indeed, it now emerges that HERV-W activation can alter glutamatergic synapse development and functions through convergent, yet different (including synergistic induction of cytokines), pathways from what was shown from previous knowledge.

Further analyses were then conducted which evidenced that neonatal ENV expression tunes glutamatergic synapse maturation and induces behavioral deficits associated to psychosis, effects which are reversed by the neutralizing anti-HERV-W ENV antibody.

The following *in vivo* study was conceived when considering that the proper maturation of glutamatergic synapses requires a switch from immature GluN2B-rich to mature GluN2A-rich synapses *(44)*. This fundamental process occurs during the postnatal period (first two postnatal weeks) in which neuronal networks and cognitive functions are established. Synaptic alterations occurring during this time window often lead to adult behavioral dysfunctions associated with psychiatric disorders, such as psychosis (*44*, *45*, *46*). Considering that the GluN2A/2B synaptic ratio was altered in vitro by ENV, we measured the GluN2A- and GluN2B-NMDAR synaptic content (Fig. 10A) at the end of the first postnatal week (P7) and at adult stages (>P65). At these stages, inserted genes were clearly present in hippocampal areas as observed by immunohistochemical and biochemical means (Fig. 10B,C). The GluN2A-NMDAR synaptic content increased over development, whereas GluN2B-NMDAR content decreased (Fig. 10D,E). However, in ENV rats the GluN2A-NMDAR synaptic content was already increased at P7 (P = 0.06), which flatten the following developmental rise from P7 to P65 (Fig. 10D). The GluN2B-NMDAR synaptic content remained stable in ENV rats compared to control rats at both ages (Fig. 10E). The relative amount of GluN2A- and 2B-NMDAR at the adult stage was thus consistently decreased (Cont: 1.0 ± 0.08, ENV: 0.90 ± 0.10, n = 8; mean ± SEM: *P = 0.034, Student's t-test). Since there was a clear sign of inflammation at P7 (not shown), we tested whether a change in the interaction between IL-1β and GluN2A-NMDA receptors (*47*) could explain the early alteration in synaptic NMDAR. The co-IP between GluN2A-NMDAR and IL1R was significantly increased in ENV-hippocampal synapses at P7 (Fig. 5F), whereas the co-IP between GluN2B-NMDAR and IL1R was unaffected. At adult stage, there was no significant difference in all groups (GluN2A/IL-1R: Cont: 1.0 ± 0.15, ENV: 1.04 ± 0.21,

GluN2B/IL-1R: Cont: 1.0 ± 0.08, ENV: 0.95 ± 0.09, n = 6-7; mean ± SEM, P > 0.05). Because ENV potentiated AMPAR synaptic content in cultured hippocampal networks (Fig. 6 G,H), we finally compared the AMPAR synaptic content at adult stages (at P7 the vast majority of glutamatergic synapses lack AMPAR). The synaptic content was significantly increased in ENV rats when compared to control ones (Fig. 5G). The overall amount of PSD-95 was unaltered in ENV rats (Fig. 5H). Thus, the neonate expression of ENV alters the developmental maturation of NMDAR subtypes with a premature increase in synaptic GluN2A-NMDAR driven by a functional interplay with the IL1-β receptor signaling complex. This accelerated maturation in NMDAR signaling potentiates glutamate synapses at a later stage, likely impairing their plastic window.

Because the maturation of glutamatergic synapses occurs during the first postnatal weeks, we postulated that the deleterious effect of ENV on the NMDAR signaling and synapse maturation is prominent during this period. To test this hypothesis, ENV rats received injections of ENV-neutralizing antibody from P4 to P12 (3 injection; Fig. 5I). At the adult stage, while the ENV-induced PPI deficit was still observed in rats that received the control antibody, the behavioral response was clearly restored by ENV-neutralizing antibody treatment (Fig. 10J).

These data indicate that ENV expression in the early postnatal period alters the synaptic GluN2A/B-NMDAR maturation, and drives the emergence of behavioral abnormalities in adults, which can be prevented and/or restored using successive *in vivo* injections of the neutralizing anti-HERV-W ENV antibody.

### EXAMPLE 4: Characteristics of the monoclonal antibody directed against HERV-W ENV protein (ENV-W2.3), which reverses and/or prevents abnormal synaptic distribution of neuronal neurotransmitter receptors GluN2B-NMDAR and HERV-W ENV-induced effects like in psychotic patients.

### 4.1 Sequences of ENV-W2.3 antibody light chain variable domain (VL).

### 4.1.1 Nucleotide sequence encoding antibody light chain variable domain

(VL)> SEQ ID NO: 12 - GN_ENV-W2.3 -VL
GATATTGTGATGACGCAGGCTGCATTCTCCAATCCAGTCACTCTTGGAACATCAG
CTTCCATCTCCTGCAGGTCTAGTAAGAGTCTCCTACATAGTAAGGGCATCACTTAT
TTGTATTGGTTTCTGCAGAAGCCAGGCCAGTCTCCTCAGCTCCTGATTTATCAGAT
GTCCCACCTTGCCTCAGGAGTCCCAGACAGGTTCAGTAGCAGTGGGTCAGGAACT
GATTTCACACTGAGAATCAGCAGAGTGGAGGCTGAGGATGTGGGTGTTTATTACT
GTGCTCAAAATCTAGAACTTCCGTGGACGTTCGGTGGAGGCACCAAGCTGGAAAT
CAAA

### 4.1.2 Amino acid sequence of antibody light chain variable domain

> SEQ ID NO: 7- GN_ENV-W2.3 -VL
DIVMTQAAFSNPVTLGTSASISCRSSKSLLHSKGITYLYWFLQKPGQSPQLLIYQMS
HLASGVPDRFSSSGSGTDFTLRISRVEAEDVGVYYCAQNLELPWTFGGGTKLEIK

**The VL amino-acid sequence along with identified CDR sequences is presented in** **Figure 11****.**

### 4.2 Sequences of ENV-W2.3 antibody heavy chain variable domain (VH)

### 4.2.1 Nucleotide sequence encoding antibody heavy chain variable domain (VH)

> SEQ ID NO: 13 -GN_ENV-W2.3 -VH
GAGGTCCAGCTGCAGCAGTCTGGGACTGTACTGGCAAGGCCTGGGGCTTCCGTGA
AGATGTCCTGCAAGGCTTCTGGCTACAGTTTTACCAGCTACTGGATGCACTGGAT
AAAACAGAGGCCTGGACAGGGTCTAGAATGGATTGGTGCTATTTATCCGGGAAA
AAGTGATACTAGCTACAACCAGAAGTTTAAGGGCAAGGCCAAGTTGACTGCGGT
CACATCCGCCAGCACTGCCTACATGGAACTCACCAGCCTGACAAATGAGGACTCT
GCGGTCTATTATTGTACAAGAACCGTCTATGCTATGGACTATTGGGGTCAAGGAA
CCTCAGTCACCGTCTCCTCA

### 4.2.2 Amino acid sequence of antibody heavy chain variable domain

> SEQ ID NO: 8 -GN_ENV-W2.3 -VH
EVQLQQSGTVLARPGASVKMSCKASGYSFTSYWMHWIKQRPGQGLEWIGAIYPGKS
DTSYNQKFKGKAKLTAVTSASTAYMELTSLTNEDSAVYYCTRTVYA
MDYWGQGTSVTVSS

**The VH amino-acid sequence along with identified CDR sequences is presented in** **Figure 12****.**

### 3.3 Complementary analyses of ENV-W2.3 antibody

VL region corresponds to a rearranged and productive IGK sequence of Kappa isotype that has no identical sequence found in databases (Uniprot databank-UniprotKB).
VH region corresponds to a rearranged and productive IGH sequence of Ig2a isotype that has no identical sequence found in databases (Uniprot databank-UniprotKB).

### EXAMPLE 5: Recognition of an epitope from two distant linear sequences on HERV-W (MSRV) protein sequence: protein conformation-induced of a conformational epitope.

### Material and methods

The experiments were performed by Pepscan, The Netherlands, as recommended by the manufacturers using ELISA plates coated with synthetic peptides overlapping over the primary HERV-W/MSRV-ENV sequence. The test antibody was diluted from 250 ng/ml to 1 µg/ml. The read-out values indicate the mean luminometric OD measured with each successive peptide.

### Results

The epitope mapping using overlapping peptides of 15 amino-acids all over HERV-W sequences (MSRV-ENV; Genbank ref. AAK18189.1 ; Locus AF331500_1 ; 542 aa) clearly identified two distant sequence stretches that were significantly detected by ENV-W2.3 antibody (Figure 13).

This clearly indicates that these sequences must be jointly detected by this antibody in the native protein, with 3D folded structure presenting these two peptide regions, **LFNTTLTRLHE** (SEQ ID NO: 10) and **LYNHVVP** (SEQ ID NO: 11), as contiguously detected aminoacids, i.e., as a conformational epitope (Figure 13).

This epitope detection characterizes this antibody, along with sequences of its CDR and of its variable light and heavy chains (VL and VH).

In conclusion, for a therapeutic application in humans, a humanized antibody or any immune-tolerated molecule is normally required to treat the patients. Therefore, the unique recognition characteristics of such humanized antibody or of equivalent therapeutic molecule, is determined by (i) CDR sequences well known to be involved in the epitope recognition and/or by (ii) the original structure of the conformational epitope joining two distant linear regions of HERV-W ENV pathogenic protein (cf. Examples 3 and 4) and/or by (iii) its ability to induce in neurons, the relocation of the NMDAR, in particular of the GluN2B-containing NMDA receptor, into the synapses.

The present invention therefore provides a therapeutic product for a novel indication in patients previously diagnosed with symptomatology- and psychologically-defined criteria, representing sub-groups of such diagnostic categories and now representing biologically defined novel entity(ies), including the one characterized by the detection of HERV-W ENV antigen in parallel with elevated levels of Il-1beta (>0.05pg/ml) and/or TNF-alpha (> 1.25pg/ml) and/or IL-6 (>0.5pg/ml) in patients previously diagnosed as "Schizophrenia" or "Bipolar Disorder". Of note, these thresholds of cytokines in serum vary with the kits, antibodies, techniques and platforms used for their dosages. Presented values are only indicative and one should adapt them according to manufacturers' instructions or assess them with a given platform. In such cases, when not having an expression confined to the deep brain tissues, HERV-W ENV antigen detection in circulating fluids may be a complementary and highly indicative biomarker.

### EXAMPLE 5: Identification of novel nosological entities including sub-groups of patients diagnosed with Schizophrenia or Bipolar disorder based on the detection of HERV-W ENV antigen and/or of other biological molecules in the serum.

### 5.1 Patients, Material and methods

Pilot studies including patients diagnosed with schizophrenia or bipolar disorder investigated current diagnostic parameters, routine laboratory biological markers and HERV-W antigen detection in the serum. Routine laboratory biological tests were performed according to manufacturer's instruction for the use of diagnostic kits, e.g., for cytokines, and, in general, according to present recommendations and regulations for medical biology analyses in France, where the analyses were performed. Concerning the detection of HERV-W ENV antigen in sera and the quantification of its circulating soluble form, all analyses were performed according to the conditions provided in the patent published under ref. WO2019201908 (A1) and entitled "Method for the detection of the soluble hydrophilic oligomeric form of HERV-W envelope protein". Results for HERV-W ENV soluble antigen in sera are expressed as "Inter-Experiment Standardized Result", corresponding to the area under the curve (AUC) of the specific HERV-W ENV soluble antigen peak calculated from the immunocapillary WES platform, normalized for inter-experiments variations using the mean+ 2 x standard deviation of series of healthy controls (lower limit of specificity/positivity cut-off value: CO) in each experiment to adjust all data to those of the first experiment used as a reference. Thus the measured AUC of each sample in each different experiment were multiplied by the ratio of corresponding CO to that of the first experiment, which adjusted all values to the same mean of non-specific background signal also taking into account the impact of inter-individual variation with twice the standard deviation as used for the determination of the minimal value of specific signal (CO). Though variations were not important, this optimized further correlation analyses with data from all subjects thereby avoiding inter-experiment slight variations to impact statistical results. Thus, all measured values were standardized with a CO of 15, above or equal to which all results indicated the specific detection and quantification of HERV-W ENV antigen. Below this CO, values correspond to non-specific background signal generated by the components of the sample and by the technical protocol with non-significant variations among negative samples ("technical noise").
To identify possible subgroups, we used unbiased two-step cluster analyses (*48*), in which we concomitantly integrated measures of HERV-W positivity (cut-off for positivity: IESR > 15; imputed as categorical variable) and serum cytokines (IL-1b, IL-4, IL-6, IL-8, TNF-a, IFN-y; all LN-transformed; imputed as categorical variables) from subjects with schizophrenia (SZ, n = 29), bipolar disorder (BP, n = 43) and matched healthy controls (HC, n = 32). Missing variables for some subjects led to a reduction in sample size. The final number of subjects included in the cluster analysis was: n(HC) = 29, n(SZ) = 18 and n(BP) = 30. The cluster analysis was run without predetermining the number of clusters, thereby avoiding bias in terms of identifying the number of possible clusters. Bayesian Criterion (BIC) was used to estimate of the maximum number of clusters, whereas the log--likelihood method was used as distance measure [1]. Following stratification of HC, SZ and BP subjects into subgroups, serum cytokines and clinical variables (age of disease onset, Montgomery-Asberg Depression Rating Scale [MADRS] score, Young Mania Rating Scale [YMRS] score, Positive and Negative Syndrome Scale [PANSS] scores, and daily chlorpromazine [CPZ] equivalents) were analyzed by one-way or two-way analysis of variance (ANOVA), followed by Tukey's post-hoc tests for multiple comparisons whenever appropriate. All statistical analyses were performed using SPSS Statistics (version 25.0, IBM, Armonk, NY, USA) and Prism (version 8.0; GraphPad Software, La Jolla, California), with statistical significance set at p < 0.05.

### 5.2 Results

Patients diagnosed with Schizophrenia (SZ), bipolar disorder (BP) and healthy controls without known psychiatric, inflammatory, infectious or metabolic disease (HC), had given informed consent to participate to a multiparametric study validated by relevant ethical committee, including clinical and treatment data in parallel with the detection and/or dosage of serum biomarkers including HERV-W ENV antigen and cytokines in their serum.
Data collected from patients and controls are presented at Figure 14.
In figure 14a, the prevalence of HERV-W ENV antigen positivity in serum is presented for HC, SZ and BP groups. A statistically significant and important difference is evidenced between healthy controls and psychotic patients either with SZ (p<0.001) or with BP(p<0.01) among which a sub-group of HERV-W ENV positive patients was identified.
These results were further analyzed for potential correlation or co-stratification (cluster analysis) with other parameters. Significant clustering was essentially found with elevated concentration of certain cytokines in sera. In Figure 14b, the predictor importance of corresponding serum values in the cluster analysis combining these parameters is presented, showing the dominant predictivity of HERV-W ENV antigen, followed by that of pro-inflammatory cytokines such as 11-6, IL-Iβ and, to a lower extent, TNF-α. Cluster analysis revealed the existence of two clusters across all tested populations, separated on the basis of HERV-W positivity and of cytokine detection in serum. Cluster 1 (CL1) represent individuals with negative HERV-W ENV antigenemia and no cytokine detection in serum (or low values within normal ranges of background detection of the corresponding technique in HC). Cluster 2 (CL2) represent individuals with positive HERV-W ENV antigenemia and significant cytokine detection in serum. In Figure 14c, the proportions of each cluster are presented for HC, SZ and BP groups. It appears that nearly all HC belong to CL1, with only one anecdotic case in CL2. Among patients diagnosed with SZ, 50% belonged to CL2 and the other half to CL1. CL2 represented about one third of all BP patients (33.3%), whereas CL1 accounted for about two third. Thus, though having a strongly significant different prevalence in psychotic patients compared to apparently healthy controls, CL2 was more represented in SZ than in BP. These results already indicate the existence of an important subgroup (CL2) of patients across SZ and BP patients presenting with HERV-W ENV positivity associated with elevated serum cytokine, whereas other patients clustering in CL1 were not different from healthy controls for these biological parameters.
Further detailed analyses of different cytokines are presented in Figure 14d. Globally, they show that only IL-Iβ, 11-6 and TNF-α serum levels from CL2 are significantly different from that of HC. Interestingly, Interferon gamma (IFN-γ) was not significantly detected. This cytokine is secreted by T-lymphocytes and is an indicator of adaptive immune responses. Therefore, in the absence of IFN-γ, the three cytokines previously shown to be elevated in CL2 are consistent with a pro-inflammatory profile caused by the activation of innate immunity. Most interestingly, IL-Iβ dominated in CL2 within BP group but not in SZ group, whereas IL-6 dominated in CL2 within SZ group but not in BP group. TNF-α was significantly different between CL2 and HC in BP group, but this was also true for CL1, and not significantly different in either cluster within SZ group. Thus, beyond the association of HERV-W ENV and pro-inflammatory cytokines detection in serum, CL2 may present different cytokine dominance between SZ and BP. The association between HERV-W ENV and IL-6 appears as a possible biomarker signature for CL2 in SZ, while the association between HERV-W ENV, IL-1β and, potentially, along with TNF-α appears as a possible biomarker signature for CL2 in BP.
Correlation and/or clustering with clinical or treatment parameters were also identified and are presented in Figure 15. In Fig. 15a, a significantly younger age of disease onset in CL2 compared to CL1 (p<0.01) is evidenced within BP group only. In Fig. 15b, a significant difference in Young mania rating scale (YMRS) is seen between CL2 and CL1 within SZ group only.
In Fig. 15c, positive and negative syndrome scale [PANSS] score sonly show a clinically expected difference between BP and SZ groups for the negative symptoms, irrespective of CL1 and CL2 sub-groups.
These data obtained with clinical rating scores used for present psychiatric diagnoses, illustrate the discrepancy between patients classification as "SZ or BP" based on clinical parameters and a classification as "CL1 or CL2 with psychotic symptoms" (to exclude HC from CL1) based on objective biological parameters causing pathogenic effects.

Finally and of major importance, in Fig. 15d, a significant difference between the daily chlorpromazine (CPZ) equivalent intake of CL2 versus CL1 within SZ group was found. This CPZ equivalent measure is a standard evaluation of the daily dose of present anti-psychotic treatment regimen of patients and, here, shows that SZ patients in CL2 require and/or are given much higher doses that SZ patients in CL1. Since other analysis excluded a possible relationship of various antipsychotic molecules in the detection of both HERV-W ENV and pro-inflammatory cytokine expression, the present observation is compatible with the fact that SZ patients with CL2 criteria have more severe and/or frequent psychotic symptoms leading physicians to prescribe higher doses to stabilize patients. Quite interestingly, this observation is also compatible with a resistance to anti-psychotic drugs in patients from CL2 group, which is also not exclusive with the occurrence of more severe and/or frequent psychotic symptoms, as previously evoked.
Therefore, though an equivalent scale for daily treatment dose evaluation in BP is not available, the more severe disease course and/or a resistance to current anti-psychotic drugs in patients with SZ with CL2 biomarkers calls for the use of other treatment strategies, implicitly suggesting to neutralize the pathogenic effects of CL2 biomarkers and preferentially those of the major predictor of CL2 (Fig. 14b), i.e. HERV-W ENV protein.
Thus, a multiparametric analysis of clinical, therapeutic and serum biomarkers data from a population comprising healthy individuals and patients diagnosed with schizophrenia or bipolar disorder, has independently led to the same evidences as those obtained from previous preclinical studies of HERV-W ENV pathogenic effects, calling for a targeted treatment neutralizing this human endogenous retroviral protein. The *in vitro* and *in vivo* efficiency of the antibody from the present invention therefore provide a unique therapeutic tool for the treatment of patients with either Schizophrenia or bipolar disorder and clustering with similar biological parameters: HERV-W ENV antigen positivity and elevated pro-inflammatory cytokine, which can be detected in blood samples, e.g., in serum (here designated as CL2). Treating patients from CL1 with this antibody is not relevant, in the absence of the targeted pathogenic protein and its elevated cytokine correlates. This implies that clinical efficacy of this antibody in patients should only be assessed in patients matching with CL2 criteria, as presently defined and not in groups of any patients diagnosed either with schizophrenia and/or with Bipolar Disorder. It may correspond to new nosological definitions or, at least, to newly defined subgroups of patients across the two types of clinical diagnoses (SZ and BP), but the most probable etiological and pathogenic heterogeneity between patients with such diagnoses is consistent with the need to define the relevant target population before applying a treatment specific for a pathogenic protein that is not involved in all cases. For routine practice and practical conditions, results from CL2 analyses also show that IL-Iβ, 11-6 and TNF-α dosages in serum may serve as surrogates for routine CL2 classification.

In conclusion, the present invention provides unexpected and meaningful evidences of (i) a previously unknown mode of action of HERV-W ENV protein on neurons relevant for psychiatric diseases, (ii) a new monoclonal antibody with novel CDR sequences targeting a conformational epitope of HERV-W ENV protein that inhibits its newly discovered pathogenic effects on neurons in an animal model of psychotic behavior/symptomatology induced by HERV-W ENV and (iii) new indications for appropriate therapeutic use of this new antibody to treat relevant sub-groups of patients, presently diagnosed with schizophrenia or bipolar disorder, with new biomarker-based criteria defining new nosological sub-groups or entities. These biomarkers, combined or alone, may therefore be used in face of relevant clinical symptoms for validating the accuracy of a treatment with this antibody in a given patient.

### References:

1. A. Sekar et al., Schizophrenia risk from complex variation of complement component 4. Nature 530, 177-183 (2016).
2. S. M. Purcell et al., A polygenic burden of rare disruptive mutations in schizophrenia. Nature 506, 185-190 (2014).
3. M. Fromer et al., De novo mutations in schizophrenia implicate synaptic networks. Nature 506, 179-184 (2014).
4. N. Grandi, E. Tramontano, Type W Human Endogenous Retrovirus (HERV-W) Integrations and Their Mobilization by L1 Machinery: Contribution to the Human Transcriptome and Impact on the Host Physiopathology. Viruses 9, (2017).
5. E. B. Chuong, N. C. Elde, C. Feschotte, Regulatory evolution of innate immunity through co-option of endogenous retroviruses. Science 351, 1083-1087 (2016).
6. R. Ono et al., Deletion of Peg10, an imprinted gene acquired from a retrotransposon, causes early embryonic lethality. Nat Genet 38, 101-106 (2006).
7. Y. Sekita et al., Role of retrotransposon-derived imprinted gene, Rtl1, in the feto-maternal interface of mouse placenta. Nat Genet 40, 243-248 (2008).
8. L. Fasching et al., TRIM28 represses transcription of endogenous retroviruses in neural progenitor cells. Cell Rep 10, 20-28 (2015).
9. R. Douville, J. Liu, J. Rothstein, A. Nath, Identification of active loci of a human endogenous retrovirus in neurons of patients with amyotrophic lateral sclerosis. Ann Neurol 69, 141-151 (2011).
10. P. Kury et al., Human Endogenous Retroviruses in Neurological Diseases. Trends Mol Med 24, 379-394 (2018).
11. M. Leboyer, R. Tamouza, D. Charron, R. Faucard, H. Perron, Human endogenous retrovirus type W (HERV-W) in schizophrenia: a new avenue of research at the gene-environment interface. World J Biol Psychiatry 14, 80-90 (2013).
12. H. Karlsson, J. Schroder, S. Bachmann, C. Bottmer, R. H. Yolken, HERV-W-related RNA detected in plasma from individuals with recent-onset schizophrenia or schizoaffective disorder. Mol Psychiatry 9, 12-13 (2004).
13. H. Perron et al., Molecular characteristics of Human Endogenous Retrovirus type-W in schizophrenia and bipolar disorder. Transl Psychiatry 2, e201 (2012).
14. D. Tzur Bitan et al., Attitudes of mental health clinicians toward perceived inaccuracy of a schizophrenia diagnosis in routine clinical practice. BMC Psychiatry 18, 317 (2018).
15. E. Vieta, M. Berk, B. Birmaher, I. Grande, Bipolar disorder: defining symptoms and comorbidities--Authors' reply. Lancet 388, 869-870 (2016).
16. I. Grande, M. Berk, B. Birmaher, E. Vieta, Bipolar disorder. Lancet 387, 1561-1572 (2016).
17. A. Lloyd, HIV infection and AIDS. P N G Med J 39, 174-180 (1996).
18. H. Perron et al., Isolation of retrovirus from patients with multiple sclerosis. Lancet 337, 862-863 (1991).
19. H. Perron et al., Molecular identification of a novel retrovirus repeatedly isolated from patients with multiple sclerosis. The Collaborative Research Group on Multiple Sclerosis. Proc Natl Acad Sci U S A 94, 7583-7588 (1997).
20. J. L. Blond et al., Molecular characterization and placental expression of HERV-W, a new human endogenous retrovirus family. J Virol 73, 1175-1185 (1999).
21. S. Levet et al., An ancestral retroviral protein identified as a therapeutic target in type-1 diabetes. JCI Insight 2, (2017).
22. R. Faucard et al., Human Endogenous Retrovirus and Neuroinflammation in Chronic Inflammatory Demyelinating Polyradiculoneuropathy. EBioMedicine 6, 190-198 (2016).
23. H. Perron et al., Human endogenous retrovirus type W envelope expression in blood and brain cells provides new insights into multiple sclerosis disease. Mult Scler 18, 1721-1736 (2012).
24. S. Noorali et al., Role of HERV-W syncytin-1 in placentation and maintenance of human pregnancy. Appl Immunohistochem Mol Morphol 17, 319-328 (2009).
25. D. J. Stein, C. Lund, R. M. Nesse, Classification systems in psychiatry: diagnosis and global mental health in the era of DSM-5 and ICD-11. Curr Opin Psychiatry 26, 493-497 (2013).
26. D. A. Regier, E. A. Kuhl, D. J. Kupfer, The DSM-5: Classification and criteria changes. World Psychiatry 12, 92-98 (2013).
27. R. K. Blashfield, J. W. Keeley, E. H. Flanagan, S. R. Miles, The cycle of classification: DSM-I through DSM-5. Annu Rev Clin Psychol 10, 25-51 (2014).
28. N. Korver-Nieberg, P. J. Quee, H. B. Boos, C. J. Simons, Group, The validity of the DSM-IV diagnostic classification system of non-affective psychoses. Aust N Z J Psychiatry 45, 1061-1068 (2011).
29. A. S. Gonzalez et al., Recombinant mutagenic 3ABC protein and monoclonal antibody for quality-control testing in foot-and-mouth disease vaccines. Antiviral Res 157, 93-101 (2018).
30. A. Rolland et al., The envelope protein of a human endogenous retrovirus-W family activates innate immunity through CD14/TLR4 and promotes Th1-like responses. J Immunol 176, 7636-7644 (2006).
31. H. Perron et al., Multiple sclerosis retrovirus particles and recombinant envelope trigger an abnormal immune response in vitro, by inducing polyclonal Vbeta 16 T-lymphocyte activation. Virology 287, 321-332 (2001).
32. L. Groc et al., Differential activity-dependent regulation of the lateral mobilities of AMPA and NMDA receptors. Nat Neurosci 7, 695-696 (2004).
33. M. B. Assie et al., F15063, a potential antipsychotic with dopamine D2/D3 receptor antagonist, 5-HT1A receptor agonist and dopamine D4 receptor partial agonist properties: influence on neuronal firing and neurotransmitter release. Eur J Pharmacol 607, 74-83 (2009).
34. R. R. Luedtke, C. Rangel-Barajas, M. Malik, D. E. Reichert, R. H. Mach, Bitropic D3 Dopamine Receptor Selective Compounds as Potential Antipsychotics. Curr Pharm Des 21, 3700-3724 (2015).
35. D. Zhu et al., Paliperidone Protects SH-SY5Y Cells Against MK-801-Induced Neuronal Damage Through Inhibition of Ca(2+) Influx and Regulation of SIRT1/miR-134 Signal Pathway. Mol Neurobiol 53, 2498-2509 (2016).
36. R. H. Johnson, D. T. Kho, O. C. SJ, C. E. Angel, E. S. Graham, The functional and inflammatory response of brain endothelial cells to Toll-Like Receptor agonists. Sci Rep 8, 10102 (2018).
37. A. Duperray et al., Inflammatory response of endothelial cells to a human endogenous retrovirus associated with multiple sclerosis is mediated by TLR4. Int Immunol 27, 545-553 (2015).
38. P. Paoletti, C. Bellone, Q. Zhou, NMDA receptor subunit diversity: impact on receptor properties, synaptic plasticity and disease. Nat Rev Neurosci 14, 383-400 (2013).
39. J. P. Dupuis et al., Surface dynamics of GluN2B-NMDA receptors controls plasticity of maturing glutamate synapses. EMBO J 33, 842-861 (2014).
40. C. A. Jones, D. J. Watson, K. C. Fone, Animal models of schizophrenia. Br J Pharmacol 164, 1162-1194 (2011).
41. M. Heine et al., Surface mobility of postsynaptic AMPARs tunes synaptic transmission. Science 320, 201-205 (2008).
42. H. Perron et al., Endogenous retrovirus type W GAG and envelope protein antigenemia in serum of schizophrenic patients. Biol Psychiatry 64, 1019-1023 (2008).
43. H. Huang et al., Chronic and acute intranasal oxytocin produce divergent social effects in mice. Neuropsychopharmacology 39, 1102-1114 (2014).
44. P. Paoletti, C. Bellone, Q. Zhou, NMDA receptor subunit diversity: impact on receptor properties, synaptic plasticity and disease. Nat Rev Neurosci 14, 383-400 (2013).
45. R. Birnbaum, D. R. Weinberger, Genetic insights into the neurodevelopmental origins of schizophrenia. Nat Rev Neurosci 18, 727-740 (2017).
46. O. Marin, Developmental timing and critical windows for the treatment of psychiatric disorders. Nat Med 22, 1229-1238 (2016).
47. F. Gardoni et al., Distribution of interleukin-1 receptor complex at the synaptic membrane driven by interleukin-lbeta and NMDA stimulation. J Neuroinflammation 8, 14 (2011).
48. T. D. Purves-Tyson et al., Increased levels of midbrain immune-related transcripts in schizophrenia and in murine offspring after maternal immune activation. Mol Psychiatry, (2019).

## Claims

1. Antibody directed against HERV-W envelope protein (ENV) for use in the treatment of a group of patients suffering from a psychotic disease and having a high level of a cytokine in a body fluid sample, in particular in a blood sample, more particularly in the serum, said cytokine being in particular a pro-inflammatory cytokine such as IL-6, IL-Iβ and/or TNF-α.

2. Antibody directed against HERV-W ENV for use according to claim 1 wherein said antibody specifically binds to the conformational epitope defined by the two distant linear sequences depicted in SEQ ID NO: 10 and in SEQ ID NO: 11.

3. Antibody directed against HERV-W ENV for use according to claim 1 or 2, wherein said antibody comprises each of the 6 CDRs as depicted in SEQ ID NO: 1, SEQ ID NO: 2, SEQ ID NO: 3, SEQ ID NO: 4, SEQ ID NO: 5 and SEQ ID NO: 6.

4. Antibody directed against HERV-W ENV for use according to any one of claims 1 to 3, wherein said cytokine is the pro-inflammatory cytokine IL-6 or the pro-inflammatory cytokine IL-Iβ.

5. Antibody directed against HERV-W ENV for use according to any one of claims 1 to 4, wherein said psychotic disease is selected from the group consisting of schizophrenia, bipolar disorder, schizoaffective psychosis and schizophreniform disorder.

6. Antibody anti-HERV-W ENV for use according to any one of claims 1 to 4, wherein said psychotic disease is schizophrenia or bipolar disorder.

7. Antibody anti-HERV-W ENV for use according to any one of claims 1 to 6, wherein said psychotic disease is schizophrenia and said cytokine is the pro-inflammatory cytokine IL-6.

8. Antibody anti-HERV-W ENV for use according to any one of claims 1 to 6, wherein said psychotic disease is bipolar disorder and said cytokine is the pro-inflammatory cytokine IL-1β.

9. Antibody anti-HERV-W ENV for use according to any one of claims 1 to 10, wherein HERV-W ENV has been detected in the patients of said group.

10. Antibody anti-HERV-W ENV for use according to any one of claims 1 to 9, wherein said antibody a chimeric monoclonal antibody.

11. Antibody anti-HERV-W ENV for use according to any one of claims 1 to 9 wherein said antibody is a monoclonal antibody or a humanized monoclonal antibody.

12. Antibody anti-HERV-W ENV for use according to any one of claims 1 to 9, wherein said antibody is an IgG.

13. Antibody anti-HERV-W ENV for use according to any one of claims 1 to 9, wherein said antibody is a humanized IgG4 monoclonal antibody or an IgG1 monoclonal antibody.

14. Antibody directed against HERV-W ENV for use according to any one of claims 1 to 9 wherein said antibody comprises:
- a light chain wherein the variable domain (VL) comprises each of the 3 CDRs as depicted in SEQ ID NO: 1 for CDR-L1, SEQ ID NO: 2 for CDR-L2 and SEQ ID NO: 3 for CDR-L3; and
- a heavy chain wherein the variable domain (VH) comprises each of the 3 CDRs as depicted in SEQ ID NO: 4 for CDR-H1, SEQ ID NO: 5 for CDR-H2 and SEQ ID NO: 6 for CDR-H3.

15. A diagnostic method to identify if a patient diagnosed with a psychotic disease belongs to a subgroup of patients suffering from psychotic disease and having a high level of a cytokine in a body fluid sample, comprising:
1) quantifying the level of a cytokine, in particular in a blood sample, more particularly in the serum, said cytokine being in particular a pro-inflammatory cytokine such as IL-6, IL-Iβ and/or TNF-α.; and
2) optionally, detecting the expression of HERV-W ENV.
